# EUROPEAN PATENT APPLICATION

(11) **EP 4 776 300 A2**
(43) Date of publication of application: **15.07.2026**
(21) Application number: 26181116.0
(22) Date of filing: 31.10.2023
(51) Int. Cl.: G16H 30/00

(54) **3D SPATIAL MAPPING IN A 3D COORDINATE SYSTEM OF AN AR HEADSET USING 2D IMAGES**

(30) Priority: 01.11.2022 US 202217978962
(62) Divisional of application: 23886654.5
(71) Applicant: Novarad Corporation, American Fork, UT 84003 (US)
(72) Inventor: CVETKO, Steven Todd, American Fork, UT 84003 (US); GIBBY, Wendell Arlen, American Fork, UT 84003 (US)
(74) Representative: ip21 Ltd

(57) **Abstract**

A method for adjusting an X-ray generated image with respect to patient anatomy using an augmented reality (AR) headset, comprises registering a position and orientation of an X-ray device in a 3D coordinate space; overlaying an X-ray generated image, as captured by the X-ray device, at the position and orientation associated with an X-ray projection field as defined in part by the position and orientation of the X-ray device and geometric attributes of the X-ray projection field, using the AR headset; receiving an identification of a 3D point of interest in the X-ray projection field from a user of the AR headset; and positioning and scaling the X-ray generated image within an X-ray projection field to include the 3D point of interest.

## Description

### BACKGROUND

Mixed or augmented reality is an area of computing technology where images from the physical world and virtual computing worlds may be combined into a mixed reality world or view. In mixed reality, people, places, and objects from the physical world and virtual constructs become a blended visual environment for users of mixed reality devices. A mixed reality experience may be provided through existing commercial or custom software along with the use of VR (virtual reality) or AR (augmented reality) headsets or devices.

Augmented reality (AR) is an example of mixed reality where a live direct view (i.e., a real view) or an indirect view of a physical, real-world environment is augmented or supplemented by computer-generated sensory input such as images, sound, video, graphics or other data. Such augmentation may be performed as a real-world location is viewed and in context with environmental elements. With the help of extended AR technology (e.g. adding computer vision and object recognition), the information about the surrounding real world of the user becomes interactive and may be digitally modified (e.g. via computer graphic overlays).

An issue faced by AR systems or AR headsets is identifying a position and orientation of an object with a high degree of precision. Similarly, aligning the position of a virtual element with a live view of a real-world environment may be challenging. For example, aligning a virtual object with a physical object as viewed through an AR headset may be difficult because the luminescence or light of the virtual object tends to obscure the underlying physical object with which the virtual object is to be aligned. Providing an approximate alignment of a virtual object with a physical object to within a few centimeters may be useful for entertainment and less demanding applications but greater positioning and alignment resolution for AR systems may be desired in the scientific, engineering and medical disciplines. As a result, positioning and alignment processes for AR used in the scientific, engineering and medical disciplines may be done manually which can be time consuming, cumbersome, and inaccurate.

### SUMMARY OF THE INVENTION

In a first broad aspect, the invention provides a method, comprising:
receiving 2D image data of patient anatomy from a medical imaging device;
identifying a location of the 2D image data in a 3D coordinate system of an augmented reality (AR) headset;
receiving a graphical mark from a user for a point within the 2D image data; and
computing a 3D coordinate of the point in the 3D coordinate system using a location of medical imaging device with respect to the AR headset to generate a 3D spatial mapping and target a structure in a person.

In a subsidiary aspect in accordance with the first broad aspect, the method further comprises displaying 2D image data while viewing patient anatomy through an augmented reality (AR) headset.

In a subsidiary aspect in accordance with the first broad aspect, the method further comprises displaying a procedure guidance indicator for a medical tool or augmentation tag identified by the AR headset to the 3D coordinate of the point.

In a subsidiary aspect in accordance with the first broad aspect, the 2D image data is an ultrasound image.

In a subsidiary aspect in accordance with the first broad aspect, the medical imaging device has an optical marker on a surface of the medical imaging device to identify a placement of the medical imaging device and provide 3D coordinates of the 2D image data.

In a second broad independent aspect, the invention provides a method, comprising:
receiving a plurality of 2D images of patient anatomy representing non-parallel projections from a medical imaging device;
identifying a position and orientation of an imaging device in a 3D coordinate system with relation to a patient and an augmented reality (AR) headset when capturing the plurality of 2D images;
receiving at least two graphical marks from a user for a common anatomical structure in the plurality of 2D images; and
determining a 3D coordinate of the common anatomical structure in the 3D coordinate system with respect to the patient by taking an intersection of projected lines from an X-ray source to the at least two graphical marks in the plurality of 2D images.

In a subsidiary aspect in accordance with the second broad independent aspect, the method further comprises displaying 2D images from the medical imaging device while viewing patient anatomy using an augmented reality (AR) headset, in order to generate a 3D spatial mapping.

In a subsidiary aspect in accordance with the second broad independent aspect, a graphical mark is at least one of a point, line, navigational track, 2D area, or 3D area target.

In a subsidiary aspect in accordance with the second broad independent aspect, the method further comprises providing a navigation path from a medical tool identified by the AR headset to the 3D coordinate of the common anatomical structure.

In a subsidiary aspect in accordance with the second broad independent aspect, the 2D images are aligned by using a virtual line placed on common anatomy in each of the 2D images.

In a subsidiary aspect in accordance with the second broad independent aspect, the 2D images are at least one of: X-ray generated images, ultrasound images, CT generated images, or MRI generated images.

In a third broad independent aspect, the method for registering X-ray generated images in 3D coordinate space while viewing a patient using an augmented reality (AR) headset, comprising:
receiving a plurality of X-ray generated images of patient anatomy;
aligning the plurality of X-ray generated images using an optically visible marker and associated image visible marker on a patient and the associated image visible marker is represented in the X-ray generated image;
receiving a first point mark-up from a user for annotating an anatomic structure on a first X-ray generated image and a second point mark-up in a second X-ray generated image, wherein the first point mark-up and second point mark-up represent common structure in the patient or a medical procedure trajectory; and
aligning the first point mark-up and the second point mark-up to generate a 3D spatial mapping using the first X-ray generated image and second X-ray generated image viewable using the AR headset.

In a subsidiary aspect in accordance with the third broad independent aspect, the optically visible marker is an optical code, a visible marker, a linear bar code, a 2D bar code, a QR code, or an April code.

In a subsidiary aspect in accordance with the third broad independent aspect, the method further comprises displaying the plurality of X-ray generated images overlaid on the patient anatomy as defined in part by alignment of the first point mark-up and the second point mark-up, using the AR headset.

In a subsidiary aspect in accordance with the third broad independent aspect, the plurality of X-ray generated images are orthogonal to each other and further comprising an additional point on a first X-ray generated image which forms a line with the first point mark-up and the additional point aligns with the line.

In a subsidiary aspect in accordance with the third broad independent aspect, the method further comprises defining positions and orientations of the X-ray generated images with respect to the patient anatomy using geometric attributes of an X-ray projection field.

In a subsidiary aspect in accordance with the third broad independent aspect, the method further comprises moving the X-ray generated images to maintain alignment with respect to the optically visible marker and associated image visible marker on the patient anatomy when the patient anatomy moves with respect to an X-ray device or the X-ray device moves with respect to the patient.

In a fourth broad independent aspect, the invention provides a method for adjusting an X-ray generated image with respect to patient anatomy using an augmented reality (AR) headset, comprising:
registering a position and orientation of an X-ray device in a 3D coordinate space;
overlaying an X-ray generated image, as captured by the X-ray device, at the position and orientation associated with an X-ray projection field as defined in part by the position and orientation of the X-ray device and geometric attributes of the X-ray projection field, using the AR headset;
receiving an identification of a 3D point of interest in the X-ray projection field from a user of the AR headset; and
positioning and scaling the X-ray generated image within an X-ray projection field to include the 3D point of interest.

In a subsidiary aspect in accordance with the fourth broad aspect, the X-ray generated image is scaled to match an anatomy scale of the patient anatomy at the 3D point of interest, as viewed through the AR headset.

In a subsidiary aspect in accordance with the fourth broad aspect, the method further comprises, modifying a position and scale of the X-ray generated image to correspond to a position and scale of the patient anatomy as viewed through the AR headset.

In a subsidiary aspect in accordance with the fourth broad aspect, the method comprises modifying the position and scale of the X-ray generated image to correspond to the position and scale of the patient anatomy further comprises overlaying the x-ray generated image at a location that is at least one of: a location intersecting a body part of a person, a location adjacent to anatomy of interest, a location in proximity to skin of the person, a location aligned with an angle of disposition of the patient anatomy in the person, or a location at a center of mass of the body part in one axis, or a location bisecting anatomy of the anatomy of the person in one axis.

In a subsidiary aspect in accordance with the fourth broad aspect, the position and orientation of the X-ray generated image is set to a position and orientation of an expected path of a medical instrument through the patient anatomy during a medical procedure.

In a subsidiary aspect in accordance with the fourth broad aspect, the method further comprises:
receiving a selection of at least one point in the X-ray generated image from a user; and
receiving a dragging instruction for the X-ray generated image in order to move the X-ray generated image.

In a subsidiary aspect in accordance with the fourth broad aspect, the method further comprises defining a geometry of the X-ray projection field using a resolution of a detector array and a distance between an X-ray emitter and the detector array.

In a subsidiary aspect in accordance with the fourth broad aspect, the method comprises:
identifying a marker with an image visible marker on anatomy of a person from which the X-ray generated image is obtained; and
aligning the X-ray generated image to the anatomy by using the marker as referenced to an image visible maker in the X-ray generated image, using the AR headset.

In a subsidiary aspect in accordance with the fourth broad aspect, the marker is an optical code, infrared reflector, or a visible marker.

In a subsidiary aspect in accordance with the fourth broad aspect, the method further comprises:
capturing a plurality of X-ray generated images from the X-ray device, wherein the patient anatomy is at non-parallel projections during capture of each of the X-ray generated images; and
positioning the plurality of X-ray generated images with respect to on another based on initial projections of each X-ray generated image with respect to the patient anatomy.

In a subsidiary aspect in accordance with the fourth broad aspect, the X-ray generated image moves when the patient anatomy moves while maintaining a projection position and orientation with respect to the patient anatomy.

In a subsidiary aspect in accordance with the fourth broad aspect, the X-ray generated image is linked to at least one of: a marker on the patient anatomy, an optical code on the patient anatomy, a morphometric model of anatomy of a person or a virtual model of anatomy of the person.

In a subsidiary aspect in accordance with the fourth broad aspect, the method further comprises:
receiving an identification of a 3D point for a corner of the X-ray generated image from the user;
adjusting a position of the corner of the X-ray generated image with respect to the X-ray device to include the 3D point; and
setting magnification or minification of portions of the X-ray generated image based on locations of the corner of the X-ray generated image with respect to a detector array of the X-ray device.

In a subsidiary aspect in accordance with the fourth broad aspect, the method comprises scaling of the X-ray generated image further comprises increasing or decreasing magnification of the X-ray generated image as the X-ray generated image is moved closer to or farther from an X-ray source of the X-ray projection field.

In a subsidiary aspect in accordance with the fourth broad aspect, the method further comprises scaling of the X-ray generated image for initial viewing using at least one of: a focal length of the X-ray device, a resolution of an X-ray receiving plate, markers on the patient anatomy of a person, a computed geometric boundary of the X-ray projection field, or optical tags on the patient anatomy of the person that determine where the patient anatomy is located in a 3D coordinate system of the AR headset.

In a subsidiary aspect in accordance with the fourth broad aspect, the X-ray device is a mini C-arm, C-arm, mobile x-ray device, angiography machine or stationary x-ray device.

In a subsidiary aspect in accordance with the fourth broad aspect, the method comprises registering a position and orientation of an X-ray device with the AR headset, further comprises registering the position and orientation of an X-ray device using a marker or optical code on the X-ray device.

In a subsidiary aspect in accordance with the fourth broad aspect, the method further comprises identifying a position and orientation of a X-ray device using a contour of the X-ray device.

In a fifth broad independent aspect, the invention provides a method for aligning an X-ray generated image with anatomy of a person using an augmented reality (AR) headset, comprising:
identifying a marker on the anatomy of the person using a sensor of the AR headset;
registering a position and orientation of an X-ray device in a 3D coordinate system;
aligning a 2D X-ray generated image, captured by the X-ray device, with the anatomy of the person based in part on at least one of: the marker, the position and orientation of an X-ray device or geometric attributes of an X-ray projection field; and
aligning the 2D X-ray generated image with the anatomy of the person using the marker on the anatomy of the person as in order to maintain alignment outside of the X-ray projection field of the X-ray device.
In a subsidiary aspect in accordance with the fifth broad independent aspect, the method comprises wherein maintaining alignment further comprises:
detecting the anatomy has moved outside the X-ray radiation field using the marker; and
re-aligning the X-ray generated image with the anatomy of the person using the marker and image visible marker on the anatomy of the person to maintain the X-ray generated image orientation with the anatomy.

In a subsidiary aspect in accordance with the fifth broad independent aspect, the method further comprises scaling the X-ray generated image when the X-ray generated image has moved away from X-ray device by using geometric attributes of a projection field stored with the X-ray generated image.

### BRIEF DESCRIPTION OF THE DRAWINGS

FIG. 1A is a diagram illustrating an example of mapping or aligning a 2D image (e.g., ultrasonic image) with a body of a person to create a 3D spatial mapping.
FIG. 1B illustrates an example of a 2D ultrasound image that may be captured for FIG. 1A.
FIG. 1C illustrates an example of a 2D ultrasound image overlaid on a body of person, using the AR headset, at a location that is defined by the location and orientation of the ultrasound transducer.
FIG. 2A illustrates an example of points of interest identified using at least two 2D ultrasonic images in order to form a trajectory or guidance path.
FIG. 2B illustrates an example where a user may sweep across an anatomical structure desired to be identified using a combination of ultrasound images.
FIG. 2C illustrates an example of two points that can be marked on two views of a common anatomical structure or location.
FIG. 3A illustrates an example arrangement of 2D images (e.g., X-ray generated images) that that may be aligned with a body of a person.
FIG. 3B illustrates an example of the 2D images that may be further aligned by using a point in the two different images of FIG. 3A.
FIG. 4A illustrates an example of a first 2D image (e.g., an X-ray generated image) of a body of the person which may be captured using a C-arm or medical imaging device.
FIG. 4B illustrates an example of a second 2D image being captured from a second position as compared to FIG. 4A by a medical imaging device.
FIG. 4C illustrates an example of a first 2D image and a second 2D image that are displayed together using an AR headset.
FIG. 5A illustrates an example of a method for displaying X-ray generated images while viewing patient anatomy using an augmented reality (AR) headset.
FIG. 5B illustrates an orthogonal side view of the first 2D image and second 2D image when they are intersected at the lines defined by the user or medical professional.
FIG. 5C illustrates example lines that may be marked in X-ray generated images with non-parallel views.
FIG. 5D illustrates an example perspective view through an AR headset of two X-ray generated images intersected at a point or line in each image.
FIG. 5E illustrates another example perspective view of two X-ray generated images intersected at line in each image, as viewed through an AR headset.
FIG. 6 illustrates an example of 2D images or X-ray generated images that may be overlaid on anatomy.
FIG. 7 illustrates an example of one 2D image or X-ray generated image that is displayed as an overlay to anatomy.
FIG. 8 illustrates an example of a medical professional guiding a needle into the simulated skull.
FIG. 9 illustrates an example of a second perspective of a user guiding a needle into a skull of a simulated patient using an AR headset.
FIG. 10 illustrates an example of a system for aligning an X-ray generated image with patient anatomy using an augmented reality (AR) headset.
FIG. 11 illustrates an example of an X-ray generated image which has been moved and scaled to match an anatomy scale of the patient anatomy.
FIG. 12A illustrates that a first 2D X-ray generated image can be a projection through an anatomical structure.
FIG. 12B illustrates an example of a second 2D image that may be captured of the anatomical structure.
FIG. 12C illustrates an example of a first X-ray generated image and a second X-ray generated image which are superimposed and displayed over anatomical structure.
FIG. 13A illustrates an example of how a first object may appear smaller in a 2D X-ray image when the image has been minified to match the size of a second object.
FIG. 13B illustrates that a corner may be moved down in the X-ray projection field and the portion of the image that captured the first object may be magnified.
FIG. 13C illustrates the use of one point in a first X-ray generated image and a line in a second X-ray generated image.
FIG. 14 is a flow chart illustrating an example method for creating a 3D spatial mapping or 3D spatial guide.
FIG. 15 is a flow chart illustrating an example of a method for 3D spatial mapping.
FIG. 16 illustrates an example method for aligning an x-ray generated image with anatomy of a person using an augmented reality (AR) headset.

### DETAILED DESCRIPTION

Reference will now be made to the exemplary examples illustrated in the drawings, and specific language will be used herein to describe the same. It will nevertheless be understood that no limitation of the scope of the technology is thereby intended. Alterations and further modifications of the features illustrated herein, and additional applications of the examples as illustrated herein, which would occur to one skilled in the relevant art and having possession of this disclosure, are to be considered within the scope of the description.

The present technology may be used to obtain one or more 2D images from medical imaging devices (e.g., X-ray devices, ultrasound devices, etc.) where a position and orientation of the images may be obtained and one or more points, lines or 3D areas may be identified on the images. These identified points may be user identified and marked points, lines, 2D areas or 3D areas, or the identified points may be machine identified points, lines, 2D areas or 3D areas. The points, lines, or 3D areas may be referenced to and added into the 3D coordinate space of an AR (augmented reality) headset. Identifying where the reference points, lines, 2D areas or 3D areas are in the 3D coordinate space of the AR headset may be calculated by determining the position and orientation of the medical imaging device which captured the 2D medical images. In addition, a depth of reference points, lines or areas with respect to a body of a person or an image source may also be determined. The user of the AR headset can then view the marked points, lines or 3D area in the 3D coordinate system of the AR headset and use the points, lines or areas for navigation (e.g., navigation of a medical instrument) during a medical procedure.

In one configuration of the technology, a plurality 2D images can be obtained and the intersection of the 2D images can be co-registered. More specifically, the position and orientation (e.g., placement) of the image views can be recorded. A point or line in the two views can then be co-registered which provides an intersection of the two 2D images at a point, a line, 2D area or 3D area. If the intersection of the two images is a line, then the line may provide a trajectory for a medical professional to navigate along or around in a medical procedure. Similarly, the medical professional may be able to navigate a medical instrument to the point or line representing the common anatomy in both 2D images. Because the AR headset records the point, line or 3D area in the 3D coordinate system of the AR headset, these reference points may be viewable in the 3D space of the AR headset. Thus, a plurality of images co-intersected by a point, a line or area may be used to navigate in augmented reality.

Previous navigation with augmented reality (AR) has used 3D datasets such as MRI (magnetic resonance imaging) or CT (computed tomography) scans. In contrast, the present technology enables more precise navigation to anatomic structures in the body of a patient using one or more 2D images.

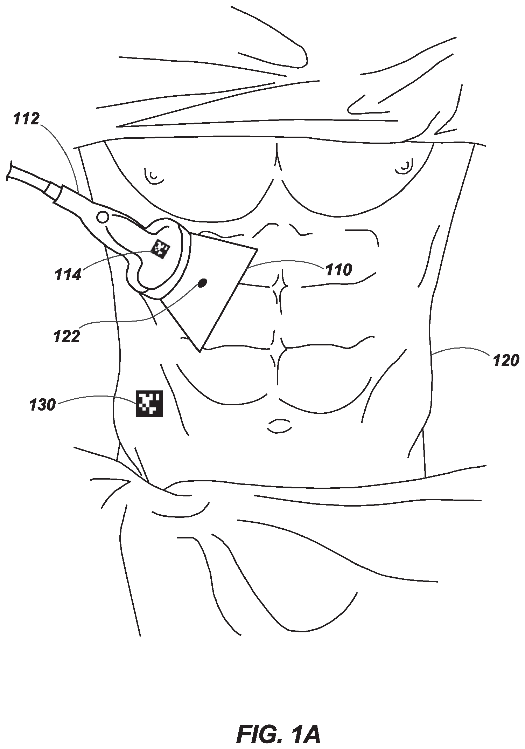

FIG. 1A illustrates a system and/or method for using the technology to create a 3D spatial mapping. In one operation, a 2D image 110 of patient anatomy 120 may be received that may represent a projection from a medical imaging device 112. The medical imaging device 112 may be an ultrasound device or another medical imaging device that provides a 2D image and includes or can determine a known depth for the 2D image.

The placement (i.e., position and orientation) of an imaging device 112 in a 3D coordinate system of an augmented reality (AR) headset may be identified by the AR headset when the 2D image is captured. The placement of the imaging device 112 may define the position (e.g., in 3D coordinate space or Cartesian space of the AR headset) and angular orientation or other spatial information about the imaging device 112. The placement of the imaging device 112 can be determined by the AR headset which can scan the optical code 114 or other marker on the imaging device 112, ultrasonic transducer or ultrasonic image capturing device.

Using one or more markers or optical codes (e.g., 2D bar codes, linear bar codes, or April codes, etc.) enables the AR headset to determine the position and/or orientation of the imaging device 112. Once the optical code 112 is captured, certain aspects about the ultrasonic image are known, including the position and orientation of the ultrasonic image with respect to the imaging device. This allows the ultrasonic image being received from the imaging device to be passed to the AR headset with this additional image information. Since the location and position of the imaging device 112 are known in the 3D coordinate system of the AR headset, the AR headset can receive the 2D image and orient the 2D image in space at the correct position and orientation at the edge of the imaging device 112 that is contacting the skin of the person.

In ultrasound imaging, the depth of anatomical structures is available from the ultrasound imaging device and the orientation and position of the image can be obtained using markers (e.g., optical codes) on the ultrasound device. With ultrasound imaging, a point in the 3D coordinate system of the AR headset can be identified with a single ultrasound scan. Ultrasound imaging can also be calibrated from the contact of the ultrasonic transducer to the skin over an anatomic area. For example, a doctor may see a vein in the ultrasound image and the vein is measured to be 10 cm deep from the transducer point on the skin as reported by the ultrasound device.

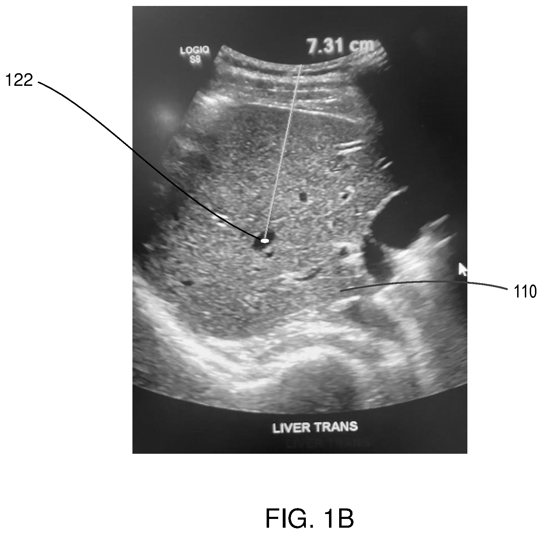

FIG. 1B illustrates a 2D ultrasound image 110 that might be captured in FIG. 1A. This image is of a liver using a curvilinear scanner. The image may be displayed to the user or medical professional in the AR headset, on a computer workstation, in a desktop computer, on a mobile device or using another display method. A graphical mark 122 can be received, as entered by a user, for a point representing an anatomical structure in the 2D image 110 from the imaging device 112. For example, a doctor may place a mark or point in the image and the ultrasound device may know or may measure the depth of the point where the graphical mark 122 is placed.

A 3D coordinate of the point may be computed for the 3D coordinate system of the AR headset using the placement of the ultrasound imaging device 112 with respect to the AR headset and a depth of the point provided by the ultrasound imaging device. The ability to define the 3D coordinate of the graphical marks can be used to generate a 3D spatial mapping. The 3D spatial mapping using the graphical mark can be used to guide a medical professional in accessing the point during a medical procedure or guide portions of a medical procedure. For example, a doctor may desire the tip of a needle or trocar to be placed at the point the doctor marked. The doctor can start to insert the needle or trocar into the body of the patient, and then the doctor may change positions or orientations of the medical tool as the doctor is inserting the needle or trocar into the body of the patient based on whether the obtained 2D image and reference point indicate that the medical instrument is on the right trajectory and may ultimately reach the marked point. The doctor can view the point from any angle using the AR headset because the point will be known in the 3D coordinate system of the AR headset. With instrument tracking, the needle or instrument can also be accurately navigated to the marked 3D coordinate.

The surface of the patient's skin may be located at the upper radius of the curvature of the ultrasonic image in FIG. 1B. Then then is a line, representing depth, draw down to the potential target or point 122. The ultrasound scanner head can have any shape, such as curved, flat, triangle, etc. An object on an ultrasound image has a depth because the transducer is contacting the patient and the time the ultrasound waves take to bounce off an anatomical object is known. When the ultrasound beam is used to capture the image, the medical professional may mark a point and the position of ultrasound image is captured with the optical code, as discussed earlier. This can result in a 3D reference point in the space defined by the AR headset. When the ultrasound device is removed, the medical professional can target that point with a medical instrument or other medical procedure.

The ultrasound images may also be linked to optical codes located on the body of the person. Then even if the patient moves, the 2D image can be moved and maintain that referencing to the person that was originally captured. For example, if a doctor needs to put a needle into a person's neck in the ICU, then the doctor can use an ultrasound to identify the point or 3D structure in the neck. The point can be displayed in the AR headset so the doctor can verify that the correct point is being reached.

The ultrasonic transducer 112 (FIG. 1A) may use a fan beam or a linear beam that is electronically guided. If the ultrasonic beam is moved or guided mechanically or electronically, this guidance can be considered when determining the location of the ultrasound image.

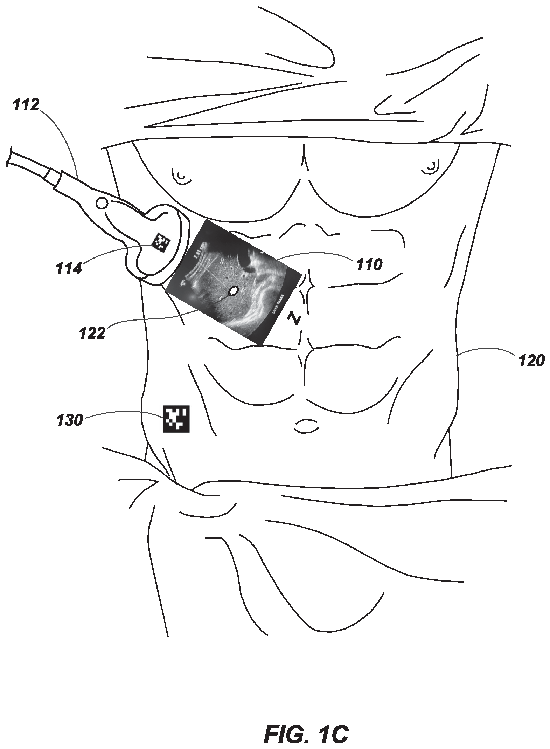

FIG. 1C illustrates an example of an ultrasound image 110 overlaid by the AR headset at a location that is defined by a position and orientation of the ultrasound transducer. The position and orientation of the ultrasound marker may be detected by an optical tag 114 (or marker) on the ultrasound transducer. For example, a medical professional may identify an anatomical structure (e.g., the center of the aorta) and mark the location 122. The optical tag 114 on the ultrasound transducer may be used as a reference anchor for the ultrasound image. The point marked by the medical professional may then be defined in the 3D coordinate space of the AR headset. This point in space may remain defined for the medical professional and the medical professional can use the point to put a needle in the patient and stick the needle in the aorta or to do a biopsy, etc. This guidance can be performed with one point on the ultrasound image, the data gathered from an optical tag on the ultrasound device and the depth of the point reported by the ultrasound device. The AR headset can detect the optical tag on the ultrasound transducer and determine a position and orientation (e.g., placement using Cartesian coordinates and angular orientation) for the ultrasound image in the 3D coordinate space of the AR headset. These operations can be performed using a single ultrasound image because the AR headset can obtain the angle, position, and depth of a point in the ultrasound image. The medical professional may navigate to the point using a medical instrument (e.g., a needle or trocar) and the AR headset can also generate a virtual portion (e.g., virtual needle) of the medical instrument as the medical instrument is being inserted in the patient by tracking the instrument.

The ultrasound image 110 may be obtained of a portion of a body of a patient using an ultrasound probe or ultrasonic transducer 112, referring again to FIG. 1A and 1B, while a medical procedure is being performed by a medical professional. The ultrasonic transducer 112 may be mobile or movable with respect to the body of the person. The ultrasound image or sonogram may be a 2D, 3D or a 4D (e.g., including a time series) ultrasound image that is produced by sound waves that bounce or echo off body tissues in a body of a patient. The echoes can be processed by a computer to create the ultrasound image or sonogram.

These ultrasound images 212 may be comparatively fast and inexpensive to obtain during a medical procedure but the resolution, accuracy and localization of the ultrasound images may not be as high as other types of imaging such as CT scans, MRIs and other imaging modalities. This technology provides the ability to locate or reference the ultrasound image 110 in the 3D coordinate system of the AR headset to assist a medical professional while performing a medical procedure. The ultrasound image 110 projected onto the patient in the AR headset may be partially transparent (e.g., using a transparency value set by the medical professional) or the ultrasound image may be opaque in the AR headset.

The ultrasound images 110 or a sonogram may also be combined with an image data set (e.g., an MRI (magnetic resonance image), CT scan, etc.) which may be more accurate, clearer, higher resolution, larger, and have better tissue contrast information as compared to ultrasound images 110. The optical codes on a person's body may be attached to an image visible marker. This image visible marker may allow the image data set that has been previously acquired from the patient to be aligned with the patient's body. Thus, a medical professional can view the ultrasound images 114 combined with a high resolution image data set, as aligned and projected in the correct position on the patient's body through the AR headset. For example, if a medical professional is performing a medical procedure on a patient's liver using ultrasound equipment, a limited portion of the patient's liver may be viewed at any one time using the ultrasound images but the entire liver may be viewed using the acquired image data set, such as CT, MRI, or PET scans, along with the ultrasound images. The ultrasound image and image data set can be co-localized in the 3D space being viewed by the AR headset. Details for using markers, optical codes and image visible markers for alignment of image data sets and images with a body of a person are described further in US. Patents 11,287,874 to Gibby entitled "USING OPTICAL CODES WITH AUGMENTED REALITY DISPLAYS"; 10,825,563 to Gibby entitled "ALIGNING IMAGE DATA OF A PATIENT WITH ACTUAL VIEWS OF THE PATIENT USING AN OPTICAL CODE AFFIXED TO THE PATIENT"; and 10,010,379 to Gibby entitled "AUGMENTED REALITY VIEWING AND TAGGING FOR MEDICAL PROCEDURES", which are incorporated by reference in their entirety herein.

In the past, ultrasound images 110 have not been anchored to a reference point relative to the patient as marked on the 2D image. Using the optical codes as described above provides a reference point within a 3D coordinate space being viewed by the AR headset. In addition, the markers (i.e., optical codes) can be used to align the ultrasound images 110 with one or more other image data sets.

If a medical professional is going to perform a medical procedure such as a breast biopsy, the medical professional may use ultrasound equipment but it may be difficult to see the actual lesion in the ultrasound image. However, the lesion may be visible in the CT, MRI, PET, nuclear, or other image data set that is displayed in combination or co-localized with the ultrasound image. Thus, the ultrasound images may be used to provide images captured (e.g., in real time) during the procedure, while the previously captured detailed anatomy may be simultaneously referenced using a higher spatial or contrast resolution image data set.

The ultrasonic transducer 114 may be passed over the surface of the body or inserted into an opening of a body. This may allow the fused or composite views of the ultrasound images and the image data sets to provide many varying composite views of a body of a patient. The alignment of the real time image with a body of a patient and image data sets may be applied to any type of real time (e.g., video) medical image where the position and orientation of the real time image may be obtained from a transducer, emitter or detector of the imaging device. An additional example of such real time imaging, which may be substituted for the ultrasound images, is CT or fluoroscopy images.

The AR headset has lenses that are semi-transparent. As a result, a medical professional who is performing a procedure may display 2D images from the medical imaging device while viewing patient anatomy through an augmented reality (AR) headset.

A procedure guidance indicator such as a graphical line can be generated by the AR headset. The line be used to guide a medical tool identified by the AR headset to the 3D coordinate of the point in the 2D image data set. The procedure guidance indicator may be other graphical user interface elements, such as graphical arrows, path indicators, animations or other guidance graphics.

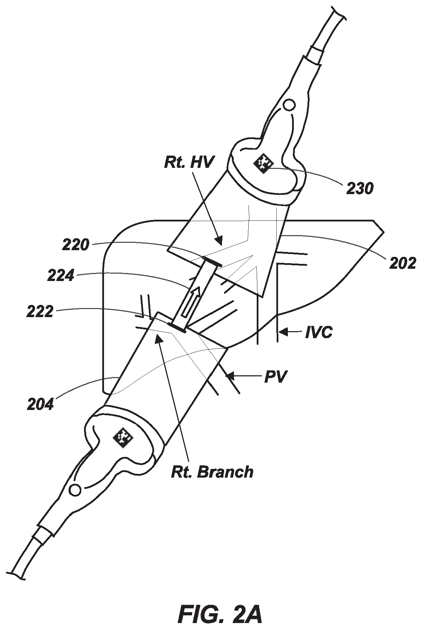

FIG. 2A illustrates an example of a first 2D image 202 that may be used together with a second 2D image 204 from an ultrasonic imaging device. A point of interest in each 2D ultrasound image may to create a guidance path 224 or track that can followed by a medical instrument controlled by a medical professional. This guidance path 224 can provide a trajectory in a 3D coordinate system of the AR headset for a doctor to follow. A first graphical mark 220 can also be placed on the first 2D image202 to mark a first point on the path, and a second graphical mark 222 can be placed on the second 2D image 204. Displaying the two ultrasound images and their respective graphical marks can help create a 3D spatial mapping (e.g., guidance path) or a 3D spatial reference guide for the medical professional while performing a procedure. Additionally, a portion of the anatomy or a cross-section of the anatomy may be imaged (e.g., a vein wall, a tube, a blood vessel, a bone, etc.) and then marked and used as guide points for the guidance path.

By obtaining ultrasound images from two different positions (non-parallel images) or two different probes, a navigation trajectory may be created. A point may be marked on each of the two or more ultrasound images and the ultrasound instrument itself has depth and directionality. The ultrasound beam may be calibrated so that the ultrasound device can provide the depth of a location in the ultrasound image. In addition, a marker or optical code 230 on the ultrasound device or the shape of ultrasound transducer may be used to determine the position and/or orientation of the ultrasound transducer. Thus, each marked point or location is known to the AR device within the 3D coordinate system. The two points can create a trajectory that can be navigated by a doctor using a medical instrument. FIG. 2A illustrates an example of creating a shunt from a portal vein (PV) to the hepatic vein (RT. HV) as in a DIPS procedure (direct intra-hepatic portacaval shunt).

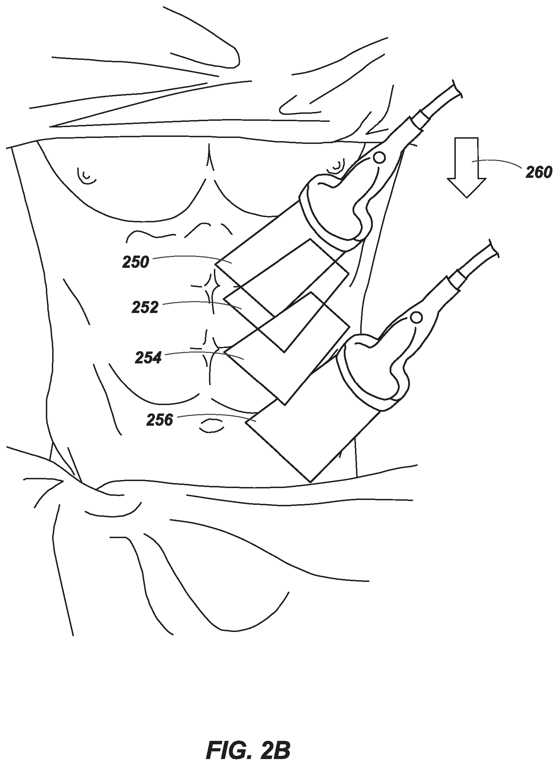

FIG. 2B illustrates that medical personnel, a doctor or a user, may sweep across an anatomical structure desired to be identified using ultrasound images. For example, ultrasound images may be used to find a vessel where a flow void that is dark can be seen or the doctor can put color on the vessel so the velocity of an arterial vessel or venous vessel can be encoded.

A portion of an anatomical structure can be identified in each ultrasound image as the ultrasound image is captured. If the medical professional sweeps the ultrasound transducer 112 along the anatomical structure, as shown by the arrow 260, then the anatomical structure (e.g., the vessel) will have multiple ultrasound images 250, 252, 254, 256 captured along that sweep. While four images are illustrated, it may be that tens, hundreds or even thousands of images maybe captured in a sweep. Accordingly, a cross-section of the tubular structure (e.g., the vessel) may be identified in each of the images.

The anatomy identified in each image may then be converted to a virtual structure representing the vessel. In other words, a virtual model may be created for the anatomy of interest, whether that is a vessel, a kidney, or a liver, etc. This virtual modeling may result in a structure in 3D coordinate space for AR headset guidance. Further, the medical professional can sweep through a portion of the anatomy to create an outline of the structure and volume of the anatomical structure. In one configuration, the medical professional can mark points on a vessel and those points can be incorporated into building the virtual model.

In one example, the virtual model may be automatically generated using automated methods. More specific automated methods of detecting the anatomical structure may include: thresholding, signal intensity detection, edge detection or artificial intelligence to detect the anatomical structure, and the system may be trained to identify specific types of anatomical structures (e.g., a vessel, a liver, a kidney, etc.).

Sweeping an ultrasound transducer across an anatomical structure may allow a user or doctor to navigate to the structure during a medical procedure following the imaging. The doctor can sweep through the anatomical structure and capture many ultrasound images, then the doctor can have AI detect the anatomical structure and build a virtual model of the anatomical structure. The system knows the depth of the anatomical structure due to the ultrasound detected depths, and the virtual anatomy that is generated can be used by the doctor to navigate with respect to the anatomical structure during a procedure using the AR headset. For example, if the doctor is going to put a needle into an artery in the groin, then the doctor may sweep the ultrasound sensor or detector along the artery. This can generate a 3D target with a shape and depth that the doctor can see through the AR headset.
Alternatively, the doctor can use marking of the ultrasound image to assist the computer AI to identify the anatomical structure.

It is useful to be able to identify a point, a line, a 2D area, or a 3D area as a target in a patient. For instance, a line may be used as a navigation path or 3D anatomy may be used to navigate to a structure or lesion or around a structure to avoid damage. When X-ray generated images are used, a plurality of images can be used to find the intersection of the images. This method includes identifying where the imaging source is or where the X-ray generated images are coming from, and this X-ray source location provides a beam trajectory. The identification of the source location and orientation can also be identified from the device itself (e.g., in DICOM information). Alternatively, the position and orientation information may be obtained from an optical code on a C-arm or gantry of the X-ray device. Furthermore, the position and orientation of the X-ray generated image can be obtained using a marker, tag or optical code with an image visible marker in the X-ray generated image and the position and orientation of the X-ray generated image can be extrapolated back to the source and may be determined using triangulation.

Once the location of image source has been identified, then a common anatomic structure or landmark may be identified in the patient. This uses the patient to provide a reference point, reference line, or reference area to align the X-ray generated images. The reference marking may be obtained from a medical professional's marking by hand, or the reference marking may be detected by a device using machine learning and machine vision. For example, a device may identify the pedicle of a vertebral bone. The marking can also be a line (e.g., an instrument trajectory), as a line is drawn along the shape of a bone or the marking can be a 3D structure.

These markings can be on two images from two non-parallel projections. The intersection of those markings can be calculated between the two X-ray generated images and this provides one or more locations in the 3D coordinate space of the AR headset. Identifying the location of the X-ray source and identifying the unifying point, line or 3D structure on common anatomy in each image, enables the computation of the 3D coordinates of the target point, line or marked area. This 3D coordinate allows targeting of the anatomic structure with a 3D guidance system in the AR headset.

The system can calculate where the X-ray source is or where the 2D image is originating from using an optical code on the imaging instrument. In another configuration, the imaging source may have a coordinate reference system (e.g., the device tells you where beam is coming from using potentiometers) using sensors to report where the imaging device is in space. Alternatively, the image visible codes can be identified on the patient and then the system can back project to the source from the image visible codes found in the 2D image(s). This technology is able to identify patient anatomy in a 3D coordinate space as viewed through an AR headset. That target may be a point, line or path, 2D area or a 3D structure.

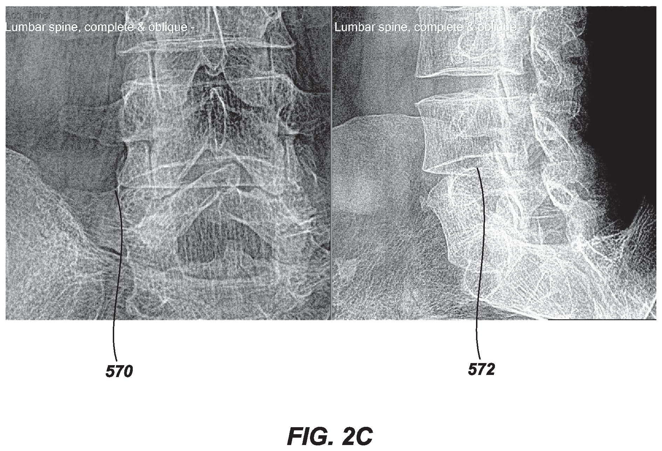

FIG. 2C illustrates an example of two points that can be marked on two views of a common anatomical structure or location (in this case the location of the medial branch). A first point 570 (e.g., represented by a circle in this illustration) that is marked in a first view of a patient's spine. This point may represent anatomy of interest to a medical professional. In addition, a second point 570 can be marked on a second view of the spine. The first point and the second point can mark a common anatomical structure. As a result, the first and second points can be aligned (as described elsewhere in this description) and for a navigation point in the 3D coordinate system of the AR headset.

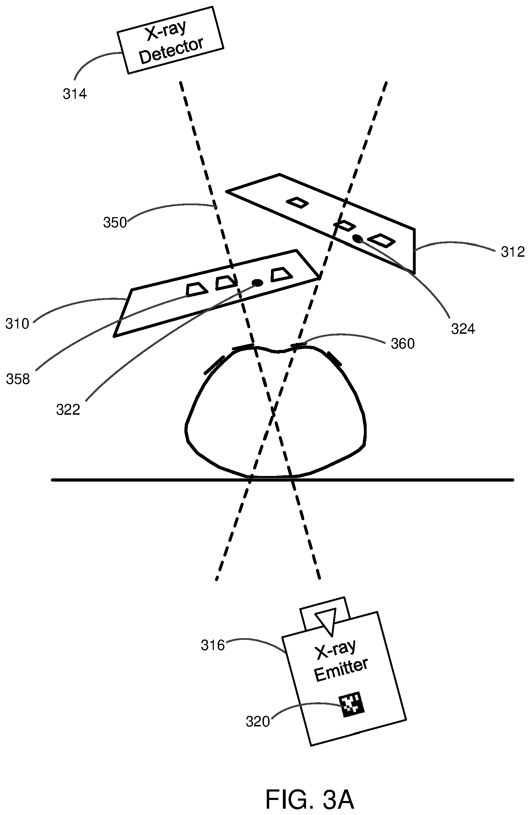

FIG. 3A illustrates an arrangement of 2D images 310, 312 that that can be aligned with a body of a person. A plurality of 2D images 310, 312 of a body or a person or patient anatomy may be received or identified. The images of patient anatomy may represent non-parallel projections from an imaging device that has an X-ray emitter 316 and X-ray detector 314. The 2D images do not have a depth associated with the image because the 2D images are projections of the body of the person and the depth of the anatomy as compared to the x-ray detector 314 may vary depending on placement of the person. In one example, the 2D images may be X-rays, ultrasound images, or other projected medical images.

Placements of an imaging device may be identified in a 3D coordinate system of the augmented reality (AR) headset when capturing the plurality of 2D images. For example, the position and orientation of an X-ray detector 314 or X-ray emitter 316 for an imaging device may be detected using pattern recognition to identify the shape of the imaging device or using at least one optical code 320 on the imaging device. The position of the device also defines the location of an X-ray emitter 316 and the path of X-rays 350 from the X-ray emitter.

At least two graphical marks 322, 324 can be received from a user for a common anatomical structure in the plurality of 2D medical images. The graphical marks may be received using a graphical interface of the AR head set. Alternatively, the graphical marks may be placed on the images using a workstation, tablet, mobile device, or other separate computing device. The graphical mark may be a point, a line, a navigational track, a 2D area, a 3D area target or another graphical mark that is on a common anatomical structure on each 2D medical image. Portions of the graphical marks can be alignable but the graphical marks are not required to be identical. A first graphical mark 322 may be on a first 2D medical image 310 and a second graphical mark 324 may be on a second 2D medical image 324. These graphical marks may be placed on common anatomical structure in each 2D image. For example, the graphical mark may be a dot that is placed on the Iliac crest, a portion of a bone, an anatomical landmark, on an air collection, on a foreign body, or on another identifiable anatomical structure that is viewable in each 2D image.

A 3D coordinate in the AR headset representing the common anatomical structure can be determined in the 3D coordinate system (or space) of the AR headset. The 3D coordinate of the common anatomical structure for the two or more graphical marks in the 2D images may be computed by extrapolating lines from a projection source (e.g., an X-ray emitter 316 treated as a point source) to the at least two graphical marks in the non-parallel projections to form an intersection of the at least two lines in order to define the 3D coordinate. The mathematical point where the two lines intersect will be the depth or coordinate in 3D space at which the common anatomical structure is located. This allows the patient anatomy to be used to co-register the two non-parallel 2D images. Then the 2D images may be overlaid at that location on the anatomical structure that represents the depth of the image as viewed by the AR headset.

Creating this overlay by displaying the two 2D images provided by the medical imaging device while viewing patient anatomy using an augmented reality (AR) headset can further generate a 3D spatial mapping. This means that a medical professional using the overlays or accurately placed 2D images may have spatial references in more than one axis for performing a medical procedure using a medical instrument on the patient. For example, a 3D spatial mapping or reference images are provided for a medical procedure in at least two axes (e.g., or in as many axes as 2D images were captured).

In addition, a navigation path that is a graphical reference can be provided from a medical tool identified by the AR headset to the 3D coordinate of the common anatomical structure. The navigation path may be a line, a curve, or another other path that can be generated and overlaid on the body of the patient using the AR headset.

Optical codes with image visible markers 460 in or on the marks can be used to identify the position and orientation of a body of a person and can also be recorded as image visible markers 358 in the X-ray image. As discussed, the image visible markers 460 can be used to align the X-ray images more accurately with respect to the patient's body or the image visible markers may be used align images from other modalities (e.g., CT, MRI, etc.) with the body as viewed in the AR headset.

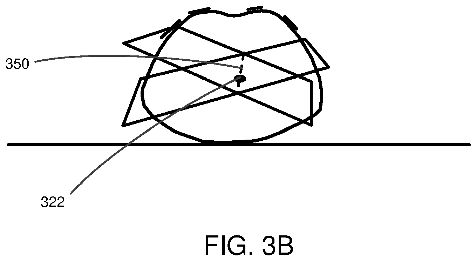

FIG. 3B further illustrates that the 2D images may be aligned by using a point in the two different images of FIG. 3A. For example, a medical professional can look at the X-ray and pin point the same anatomical spot or a corresponding point of interest on two X-ray generated images. Both images can be in different planes. For example, the images may be perpendicular or at another angle to each other. So, this anatomy is calculated to be at a specific point in 3D space with respect to the AR headset. The medical professional can then guide a needle to the point using only two 2D X-ray generated images. Each X-ray generated image may be in its own virtual pyramid that represents the projection of X-ray generated images. In addition, a virtual line 350 can be placed on common anatomy in each of the 2D images. The 2D images may be set to intersect on that line or another line defined by the user that is in the same position in each 2D image. As can be seen in FIG. 3B, the 2D images may intersect at an angle and this difference in angle between the 2D images may result in the 3D spatial mapping that can be viewed by the medical professional. For example, a doctor may use a needle in a medical procedure and the doctor can see when the needle is moving up and down toward the right point or line in the planes. Two points of interest may create a guidance path or track that can followed by a medical instrument. This line can provide a trajectory in 3D for a doctor to follow. The 2D images may be X-ray generated images, ultrasound images, CT generated images, and MRI generated images.

This aspect of the technology can use two planar images and find a 3D target in space for the AR headset. The patient's own anatomy can be used by a medical professional or machine methods to co-register the images. The co-registration can be performed by obtaining spatial information from the image source to be able to align the images to the spatial information known about the patient in the 3D coordinate system of the AR headset. The use of more than one image view for X-ray generated images (or images without a measured depth) provides 3D navigation.

The images can be an X-ray, ultrasound image, fluoroscopic image or another planar medical image. On the images of a patient, a common anatomy can be identified in the two images that are non-parallel projections. The patient anatomy can be identified using a point, a line, 2D area, or a 3D area.

The images can be referenced in terms of their spatial localization using different methodologies. One method may use image visible codes on the skin of a person, and this provides some of the spatial information. Then information can be provided by a user or image detection regarding how the two images are related to each other. If the images are at 90 degrees or 30 degrees with respect to each other, then a change in angles between the images also changes where the intersection point is between the images. Thus, the orientation angular data may be from an image visible tag on the patient, and the image visible tag can be used calculate the angle at which the image was captured by the medical image device.

In another method, an optical tag may be on the X-ray imaging device or fluoroscope device and the optical tag can provide a reference for the position and orientation of the X-ray imaging device with respect to the patient. In another method, the C-arm of a fluoroscope (e.g., for angio imaging) may have an angle provided from an internal sensor and then the angle can be coded into the DICOM data representing the projection angle of the capture image, and the device may transfer this position and/or orientation information over the local network to the AR headset or by other means communicate to the AR headset. This information may indicate how the plurality of images are interrelated.

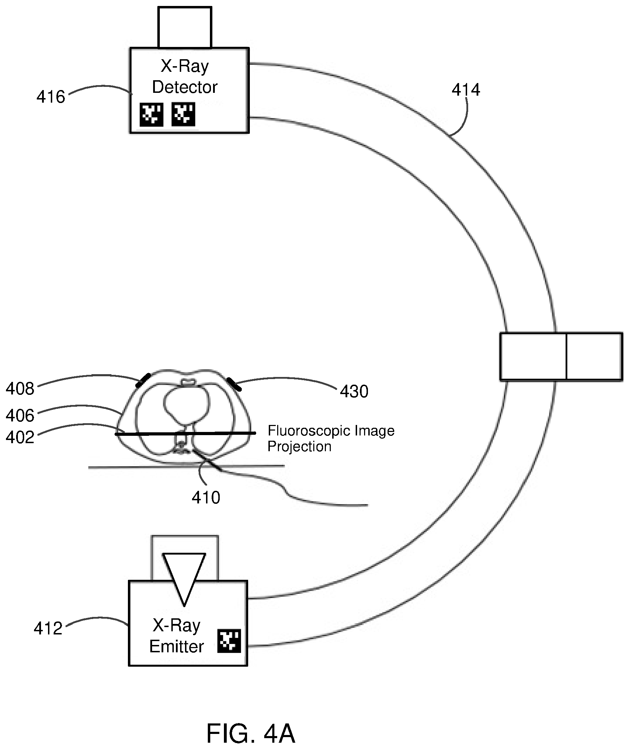

FIG. 4A illustrates a first 2D image 402 (e.g., an X-ray generated image) of a body of the person 406 can be captured using a C-arm 414. The C-arm may have an X-ray emitter 412 and an X-ray detector 416. The person's body 406 may also have optical codes 408, 430 with image visible markers and the 2D images may be linked to the optical codes and if the person's body moves with the optical codes then the optical codes and image visible codes can be used as reference for moving the 2D images in the display of the AR headset. A medical instrument 410 may be used during a medical procedure and may be included in the 2D image 402.

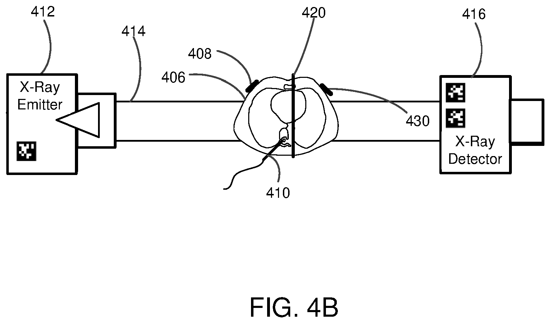

FIG. 4B illustrates a 2D image 420 being captured from a second position by a medical imaging device. For example, the medical imaging device may be the C-arm 414 capable of capturing X-ray generated images. The medical imaging device may have an X-ray emitter 412 and an X-ray detector 416 that are set to a different position and/or orientation to capture an additional 2D image or additional 2D X-ray projection as compared to the first orientation of the C-arm (FIG. 4A) used to capture the first 2D image of the body of the person 406.

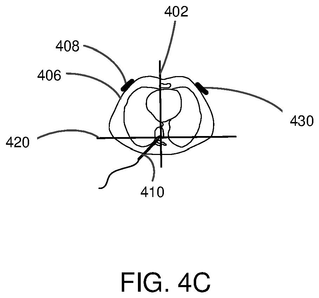

FIG. 4C illustrates an example of a first 2D image 402 and a second 2D image 430 that are displayed together using an AR headset. The 2D images are displayed with the same position and orientation to a body of a patient as the position and orientation at which the 2D images were captured. The first 2D image 402 may be at a 90 degree angle from the second 2D image 420 in this example. However, any angle may exist between the first 2D image 302 and second 2D image 420, as defined when the 2D images are separately captured. As mentioned earlier, a 3D spatial mapping or reference images can act as guides for a 3D coordinate space in at least two axes during a medical procedure. A medical professional can guide a medical instrument 410 using the 3D spatial mapping and can view the 3D spatial mapping which can help guide the medical professional as to where the instrument is with respect to the tissue of the body of the person or patient.

To reiterate, some of the configurations described earlier can use at least two images that may be: X-ray generated images, fluoroscopic images, ultrasound images, angio images, real-time X-ray video, etc. Both the X-ray device and the ultrasound transducer can have one or more optical tags on the device that gives their relative position and orientation in space when a 2D image is captured. However, if two non-parallel X-ray generated images are used, then no codes are required on the X-ray device or ultrasound transducer because the codes on the patient allow us to triangulate and determine the beam of the X-ray. Then a common anatomic structure may be identified using a point, a line, two lines, a 2D area, or a 3D area on each 3D image. The intersection of the two images provides a 3D coordinate structure in the AR headset that a medical professional may navigate to or navigate around.

A medical professional may obtain two X-ray generated images of a patient and those X-ray generated images can reference the image visible codes that are on the patient. This reference may indicate where the 2D images are relative to the patient. If only a single point is marked on each X-ray generated image, a medical professional can navigate to that point in space. However, a full intersection of the images may not be obtainable, but the single common anatomical location may be correctly viewed through AR headset. This method may provide a single point target in the 3D coordinate space of the AR headset, even if the images might not be aligned. On the other hand, if the medical profession desires to navigate along a trajectory then a line can be used in both 2D images.

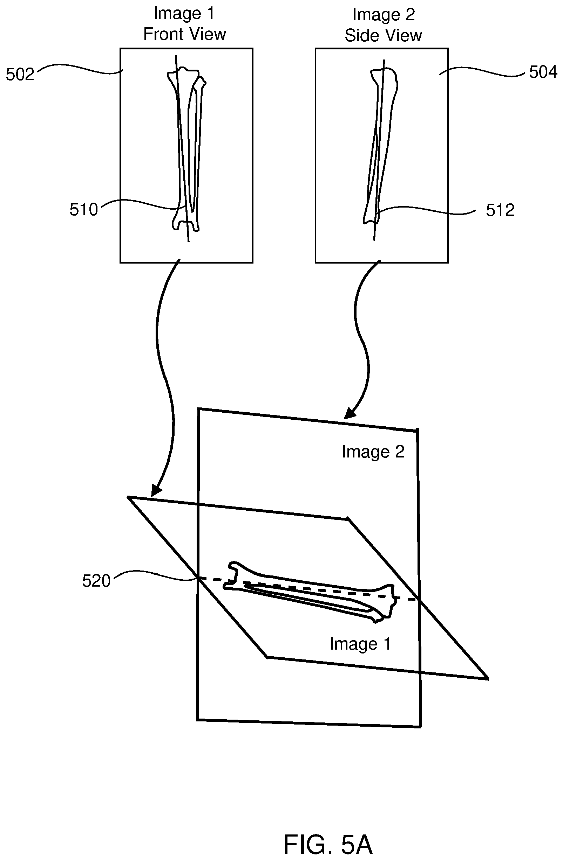

FIG. 5A illustrates an example of a method for displaying X-ray generated images 502, 504 while viewing patient anatomy using an augmented reality (AR) headset. A plurality of X-ray generated images of patient anatomy may be received from an X-ray device. Each of the X-ray generated images may represent non-parallel placements of patient anatomy relative to the X-ray device or non-parallel placements of the X-ray device with respect to the patient anatomy. Each non-parallel placement may define non-parallel positions and orientations of the patient anatomy with respect to the X-ray device or vice versa.

A first line mark-up 510 may be received from a user for a first X-ray generated image 502, and a second line mark-up 512 using at least one point marking in a second X-ray generated image 504 may also be received. The mark-up can represent common anatomical structure between the first X-ray generated image 502 and second X-ray generated image 504.

In one configuration of the technology, one line may be drawn on the first image and the second line may be created by using one point marked on the second X-ray generated image. This one point on the second X-ray image may form a second line by using a view point of the AR headset. This second line that is formed by implication can then be aligned with the first line to provide an intersection between the two 2D images.

The first line 510 and the second line 512 can be aligned to generate a 3D spatial mapping using the first X-ray generated image 502 and second X-ray generated image 504 visible using the AR headset. The dotted line 520 illustrates the line where the first line 510 and the second line 512 can be aligned for the common anatomical structure. In other words, the line can be drawn in the same location on each image using the common anatomical structure as a reference. This line is in both images and may be used by a medical professional, such as a doctor performing a procedure, to guide where the doctor is placing a medical tool. For example, the medical professional may follow the line as a guide path for performing the procedure (e.g., inserting a needle). The medical professional knows that when the line is being followed, the medical procedure is likely proceeding accurately. The medical tool may be a needle, a trocar, an endoscopic tool or other medical tool.

Thus, the medical professional can have a 3D spatial reference for using a medical tool or for identifying where incisions are to be made using at least two axes. This 3D reference is provided while only capturing a limited number of 2D images (e.g., 2+ images from different projection points or different perspectives). This means the patient does not need to have CT scan or MRI prior to a medical procedure but only a limited number of X-ray image captures may be used. This may reduce the amount of exposure the patient has to X-rays, magnetic fields, medical dyes, etc.

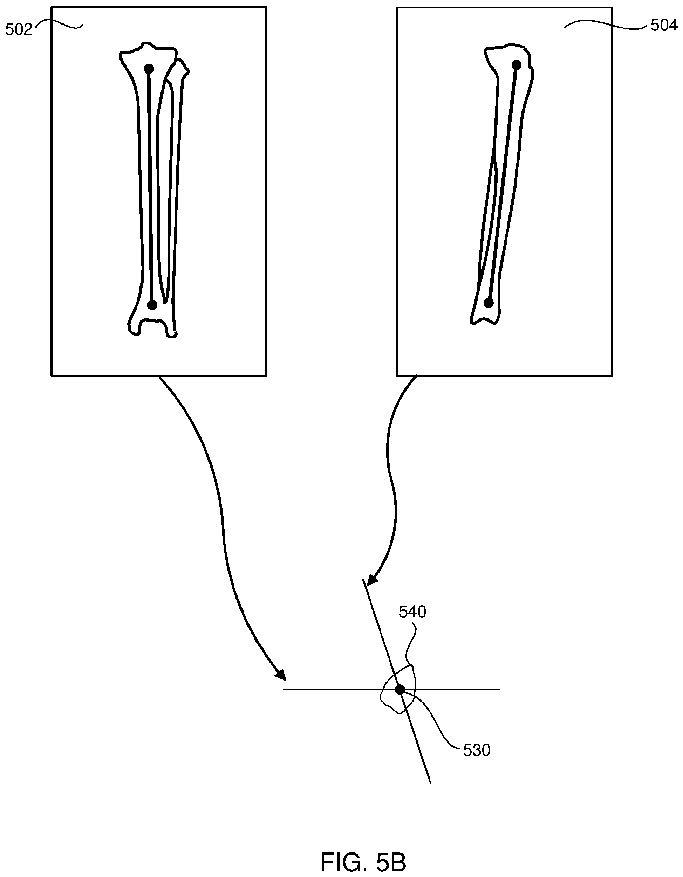

FIG. 5B illustrates an orthogonal edge view of the first 2D image 502 and second 2D image 504 when they are intersected at the lines defined by the user or medical professional. The dot 530 represents an edgewise view of both lines that are intersected and as they are viewed with both the 2D images edgewise to an AR headset or the user. The outline 540 of a cross section representing where the patient's bone may be can also be seen in FIG. 5B. This cross section of the bone may not be visible to a user in practice but it represents how the 2D images may be aligned with the real anatomy using the AR headset. In another example, a doctor could also navigate the length of the bone, for example, in order to place an intramedullary rod.

The plurality of X-ray generated images may also be aligned with the patient anatomy visible through the AR headset using at least one optical code and an associated image visible marker represented in the X-ray generated image. Thus, the position and orientation of the X-ray generated image may be partially defined by the position and orientation of the optical code and image visible marker that can be seen by the AR headset and in the X-ray generated image.

The 2D images may be linked to the movement of the patient anatomy visible to the AR headset using optical codes and associated image visible markers. As a result, the X-ray generated images may be moved to maintain alignment of the 2D images with respect to one or more optical codes and image visible markers on the patient anatomy when the patient anatomy moves away from the X-ray device. For example, once the 2D images are linked to the optical codes, then when the patient moves their arm away from the X-ray device, the AR headset can maintain the display of the 2D images with respect to arm that has been moved. The 2D images can maintain their previous orientation and position with respect to the patient's arm being moved by using the optical codes and image visible markers. This allows to a medical professional to observe the X-ray generated images in the correct position with respect to the arm, even when no X-rays are being used and no X-ray generated images or video are actively being captured.

In one configuration, the orientation and/or position of the 2D images may be defined by the position and orientation of the imaging device that is capturing the 2D images. For example, the shape of the imaging device may be detected and the X-ray source of the images may then be known. For example, the position of the X-ray source may be computed using the AR headset. As a result, the way in which the 2D images should be oriented and/or positioned may also be known. Similarly, one or more markers may be on the imaging device and this may provide the position and orientation of the imaging device and in turn provide the position and/or orientation of the 2D images. The markers may be optical codes or other visible markers.

The positions and orientations of the X-ray generated images may also be defined with respect to the patient anatomy using geometric attributes of an X-ray projection field.

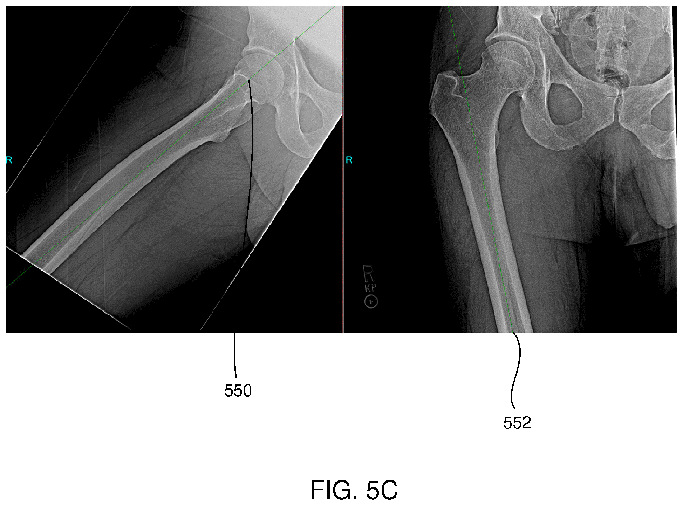

FIG. 5C illustrates that a line for anatomy 550 may marked on a first X-ray generated image. In addition, a second line 552 for common anatomical structure may be marked on a second X-ray generated image. These two lines may be aligned as discussed and illustrated in FIGS 5A and 5B.

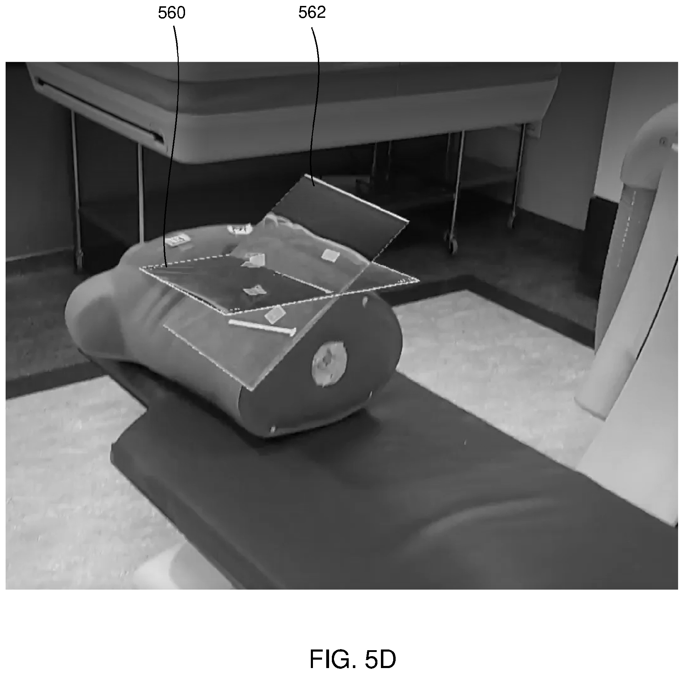

FIG. 5D illustrates a perspective view of two X-ray generated images intersected at a point or line in each image, as viewed through an AR headset. The first X-ray generated image 560 and the second X-ray generated image 562 are overlaid on a simulated patient or X-ray phantom. The first X-ray generated image 560 and the second X-ray generated image 562 may be intersected at a line or point. In this example, the medical professional may use the intersection of the two images as a guidance track for a medical procedure. For example, a needle may be guided down the line at the intersection of the two images, or a needle may be guided to a point (or other marks) in the 3D coordinate space of the AR headset.

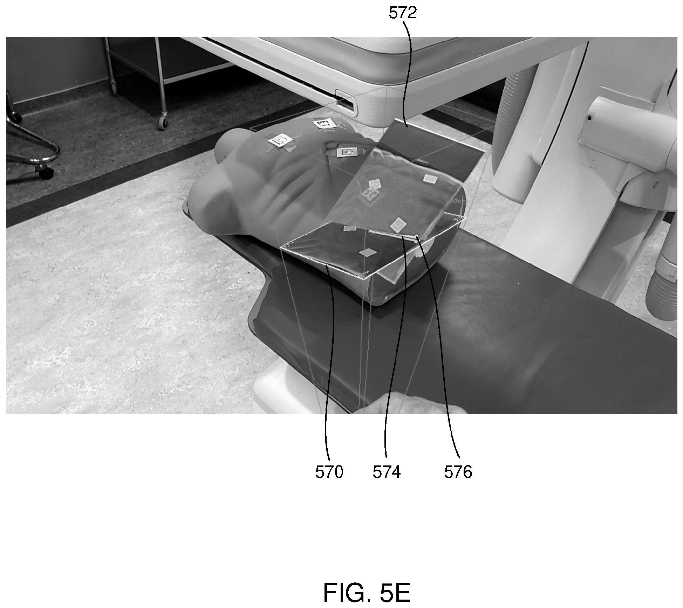

FIG. 5E illustrates a perspective view of two X-ray generated images intersected at a line in each image, as viewed through an AR headset. The first X-ray generated image 570 and the second X-ray generated image 572 can overlaid on a simulated patient or X-ray phantom. The first X-ray generated image 570 and the second X-ray generated image may intersected at a line 574. In this example, the medical professional may use the intersection of the two images as a guidance track for a medical procedure. For example, a needle 576 may be guided down the line 574 at the intersection of the two images. The line 574 on each 2D image may be a line that is formed on a common anatomical structure such as specific bones or tissues and thus the 2D images can be matched or intersected at that line.

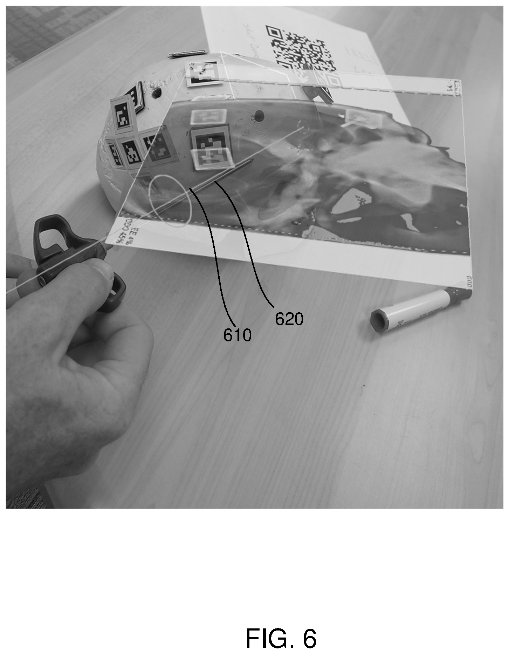

FIG. 6 illustrates that the plurality of 2D images or X-ray generated images may be overlaid on anatomy (e.g., in this case an example skull). However, in FIG. 6 only one X-ray generated image is shown at a time. The overlay of the two or more 2D images may be defined in part by alignment of the first line and the second line 610, using the AR headset. The one 2D image that is being shown is overlaid on a test skull and the user is following the line with a needle or trocar 620 simulating a medical procedure.

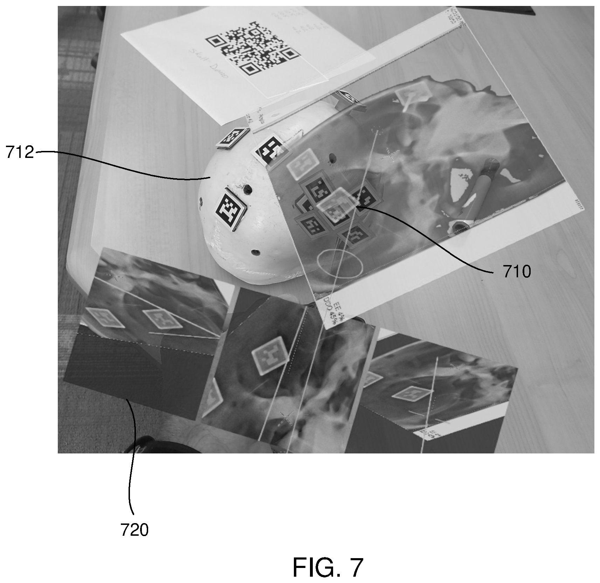

FIG. 7 illustrates one 2D image or X-ray generated image that is displayed as an overlay to anatomy 712. A heads up display of multiple breakout views 720 of the X-ray generated images is also displayed. The breakout view may show X-ray generated images using navigational views desired by a medical professional (e.g., orthogonal to the anatomy, etc.) with the X-ray generated images as overlays. They are navigational views illustrating the medical device line and or graphical markings as compared to other X-ray generated image views or other perspectives viewing the overlaid X-ray view. For example, coronal, axial and sagittal views, etc. may be provided.

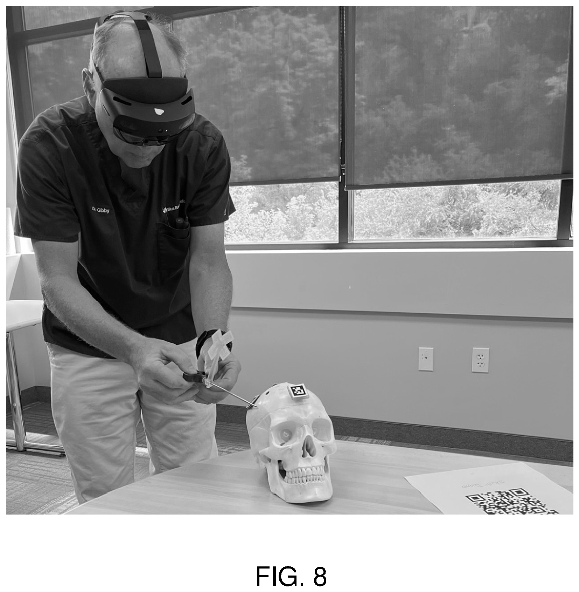

FIG. 8 illustrates a medical professional simulating the guidance of a needle into the example skull. In order to obtain the images or video, an X-ray intercept box may intercept the X-ray generated video or stills and route the video or still images to the AR headset of the doctor rather than sending the images to a computer monitor. The AR headset may then have real time X-ray generated video, and the large monitors in the operating room may be avoided. A still snapshot of the X-ray generated video can be taken at a point in time or a still image may be extracted from the video by the AR headset. The QR code by the anatomy (e.g., off to the side of the skull in FIG. 8) can provide information to enable the AR headset to get the live video using an IP address of the X-ray intercept box that is obtained from the QR code.

The doctor may perform an initial room mapping by looking around the room. Then the doctor may look at each optical tag from several angles to enable the AR headset to register the location of each optical tag. The optical tags may have an image visible marker that is metal under the optical tag (e.g., titanium or stainless steel). The image visible markers may be visible in the X-ray generated images. X-ray generated images may be captured from at least two different angles. The X-ray generated image capture from a specific view is tied to the optical tags that are visible. The AR headset may also provide a virtual monitor in the top of the doctor's AR headset view that is a live view and changes based on current incoming images from the X-ray device. A foot pedal may be used to start and stop x-ray exposures.

In one example, two different X-ray generated images may be obtained at two different or non-parallel angles but only one X-ray generated image may be shown at a time based on the angle of the doctor or viewer. For example, the X-ray generated image most perpendicular to the viewer or doctor can be shown. Alternatively, two more X-ray generated images may be shown at the same time. The X-ray generated images may be semi-transparent or they may not be transparent. In one configuration, a doctor can toggle between the two or more different projections provided by the X-ray generated images. The X-ray generated images can be lined up so they are in plane with the anatomy of interest, and the images can intersect at anywhere between just one or two degrees on up to 90 or more degrees. The doctor or user can pinch the X-ray generated images to move the image along the X-ray projection field (e.g., along a ray in the virtual cone or virtual pyramid) through the anatomy so the X-ray generated image is sized correctly, as will be discussed more later.

A point or line representing common anatomical structure (e.g., a common anatomical point or linear structure) may be made by a doctor. The X-ray generated images may be intersected at that point or line. A needle can be guided by the doctor to the point shown in both X-ray generated images or doctor may control the needle to follow the line where both X-ray generated images intersect. Thus, there may be a virtual track through the body that hits the point in the 3D space of the AR headset. The line may be created though two anatomical points in each X-ray generated image, and the planes of the X-ray generated images can intersect along the line. The doctor can then navigate to the desired point or along the line with just two X-ray generated images while viewing the anatomy from different angles or perspectives (depending on the doctor's head position). Accordingly, the needle can go through the same two points or along the same line in both X-rays. For example, the needle can move along the same part of a bone in both X-ray generated images.

The doctor can pass through desired point or line without a significant amount of advanced planning. The doctor can simply put the optical codes with metal tags on the patient and start the X-ray process. This technology enables the use of X-ray generated images for real-time guidance during a procedure. A doctor can take an X-ray and say, "The medical tool positioning looks good from that angle", but then take another X-ray and check from another angle. Then if the medical tool is off target, the doctor can take another X-ray generated image. With this method a limited amount of X-ray generated images (e.g., 2 or 3) are obtained to check that the correct anatomy is being accessed in a procedure. This is in contrast to taking 40+ X-rays as a procedure progresses without using the X-ray generated images in an AR headset to create a 3D guide.

The X-ray generated images may be at any angle in an X-ray field or pyramid and not just orthogonal to the X-ray source and the system can extrapolate the X-ray generated image to the desired size. A doctor can adjust the size and angle of the X-ray to make the X-ray images align by hand, if desired. An X-ray beam or X-ray radiation field is generally considered to be a cone shape but the X-ray generated image is rectangular due to the shape of the x-ray sensor.

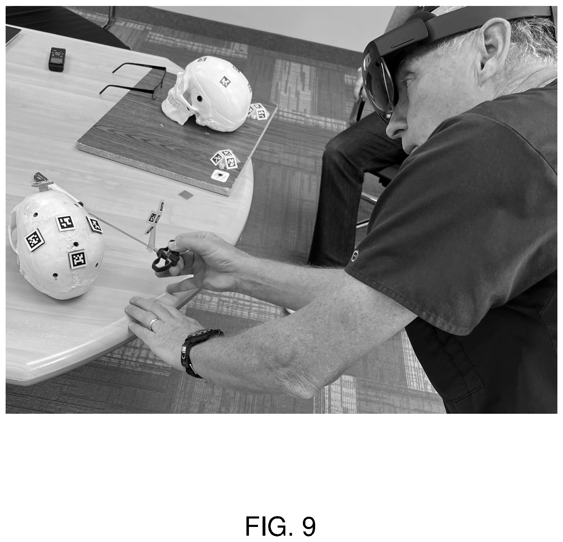

FIG. 9 illustrates a second perspective of a doctor guiding a needle into a skull of a simulated patient using an AR headset. In one configuration, X-ray video may be used. In addition, a radiopaque instrument can be seen inside the body in real-time on the X-ray video. The AR headset can see or register the C-arm position and orientation with the emitter (e.g., a top of the pyramid) and the detector (e.g., the bottom of the projection field or pyramid) and an optical code on the person. When the doctor drags the X-ray into the middle or core of the subject anatomy, then the X-ray generated images can be sized to match the anatomy. The final result may be a live X-ray generate image with accurate scaling for the anatomy that is overlaid on a patient through the AR headset, and the medical instrument may be visible as the medical procedure occurs. A similar result can also be provided with a series of still X-ray generated images.

In one configuration, when the X-rays are actively being projected, a colored (e.g., red) border, virtual rays, or wavy lines may be shown around the video or wavy lines may be shown coming down from the X-ray emitter to warn of X-ray exposure. In addition, the AR headset can show how much x-ray scatter there is and where. These notifications may be shown to the doctor in the headset (in real time) when the x-ray machine is currently active (emitting radiation). Further, a sound could be utilized as feedback to alert the doctor to the active generation of X-ray radiation.

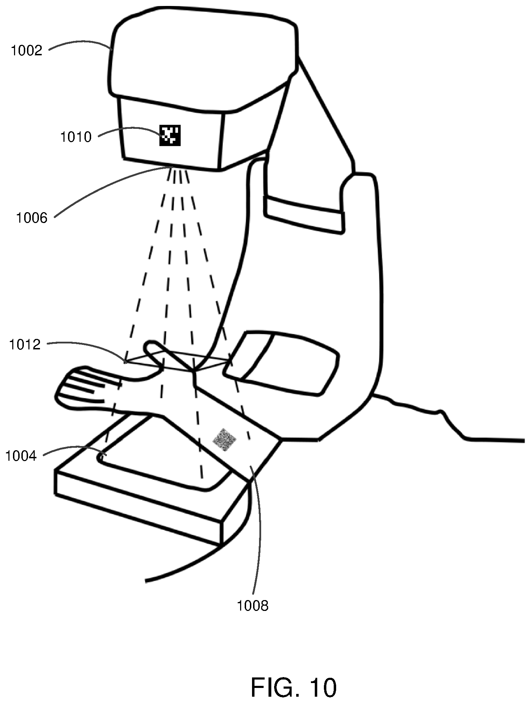

FIG. 10 illustrates a system and method for scaling and aligning an X-ray generated image with patient anatomy using an augmented reality (AR) headset. The technology may use an imaging device 1002 that is a smaller portable C-arm which includes an X-ray detector 1004 plate and X-ray source 1006. The imaging device 1002 may be on a cart with wheels, and a smaller imaging device may be made for use in imaging legs and arms and anatomical extremities. For example, a patient's arm 1008 can be imaged in the portable C-arm in an operating room, and then the patient can be taken a nearby operating table to perform a medical procedure on the arm 1008.

A position and orientation of an X-ray device 1002 may be registered with an AR headset. The X-ray device may be detected: by using a marker, by identifying an optical code 1010 on the X-ray device or by detecting the shape or contour of the device using pattern recognition. The X-ray device may be a mini C-arm, C-arm, mobile x-ray device or another semi-movable X-ray device. For example, one or more markers, optical codes, QR codes or April codes 1010 may be placed on the mini C-Arm. The optical code may be used to link the AR headset to a live view of what the X-ray device is capturing, and this linking may use an image router to which the AR headset connects. For example, the live X-ray generated image or X-ray video can be oriented in the 3D coordinate space based on the position and orientation of the X-ray device. In addition, the live or still X-ray generated images can also be shown a flat computer screen nearby.

Live X-ray generated video can be streamed to the AR headset and stills of the X-ray video may be captured using voice commands, virtual controls or physical controls connected to the X-ray device or the AR headset. The user can also crop away part of a still X-ray generated image that was captured, if desired. When the AR headset is used, the X-ray generated image can be shown on the waveguide or display glasses of the AR headset, as being located at the area where the X-ray generated image is taken. This may be provide a view that appears to be within the projection field (e.g., X-ray pyramid) for the X-ray radiation area. The QR code 1010 or another optical code can provide positional information for the X-ray device so that the AR headset can detect where the X-ray detector is in 3D space.

The X-ray generated image can be displayed on the X-ray detection plate or anywhere in the X-ray projection field as long as the X-ray generated image is scaled for that point in the X-ray projection field. An X-ray generated image 1012, as captured by the X-ray device for a patient, may be overlaid at a position associated with an X-ray projection field as defined in part by the position and orientation of the X-ray device and geometric attributes of the X-ray projection field, using the AR headset. The geometric attributes may include the attributes of the X-ray image that allow the X-ray image to be scaled including: the focal-distance (from source to detector of the X-ray device), and the physical size of the x-ray detector. The resolution of the image may be used as a geometric attribute and it may be computed from information known about the system. With this information, and the registration to the patient anatomy, the X-ray image can be displayed in any position in the projection field or along a ray from the X-ray source to the center of the X-ray detector when the x-ray was taken for the patient anatomy. As the x-ray is moved from the source to the detector the X-ray generated image can be magnified, starting at a tiny point, unit the X-ray generated image is the size of the detector. This forms a "pyramid" or "cone" for the projection field or radiation field.

A user of an AR headset may identify a 3D point of interest in the projection field. The X-ray generated image may then be scaled within an X-ray projection field to include the 3D point of interest. In an alternative configuration, a move instruction may then be received from a user of the AR headset to move the X-ray generated image to a location that includes a 3D point of interest between a detector array and X-ray emitter. The move instruction may be a drag instruction for the X-ray generated image. Alternatively, the move instruction may be performed using a cursor or graphical buttons displayed on the user interface of the AR headset.

In one example, the user may move one or more corners of the X-ray generated image. When the corner point is moved, the scale and position of a portion of the X-ray generated image may be modified within an X-ray projection field based in part on the position for the point in the X-ray generated image as associated with a geometry of the X-ray projection field. A geometry of the X-ray projection field may be defined using a resolution of the detector array and a distance between the X-ray emitter and the detector array.

In some uses, the distance between the X-ray emitter and detector array may not need to be known. If the anatomical target is in the center of the x-ray beam, then the intersection of the points can be found accurately without knowing the distance between the X-ray emitter and detector array. However, if the anatomical target is not in the center of the X-ray beam (which is most of the time), then a geometrical distortion effect can occur. Knowing the distance between the X-ray emitter and the detector array can enable the projection distortion to be accounted for in the depth computations. Marking or detecting common anatomic structure can also assist with such depth calculations. The geometry cannot generally be computed unless the anatomy is centered. However, if the distance from the source to the detector is known then the geometry of anatomy that is off axis can be determined.

In another example, the user interface of the AR headset may receive a selection of a corner of the X-ray generated image from the user. Then a move instruction may be received from the user for the corner. A position of the corner of the X-ray generated image may be adjusted with respect to the X-ray device (and secondarily the AR headset). Then magnification or minification of portions of the X-ray generated image may be set based on the locations or distances of each corner of the X-ray generated image with respect to the detector array of the X-ray device. The scale of the X-ray generated image may be modified by increasing or decreasing the scaling of the X-ray generated image as the X-ray generated image is moved closer to or farther from an X-ray source of the X-ray projection field. The scaling of the X-ray generated image for initial viewing may use at least one of: a focal length of the X-ray device, a resolution of an X-ray receiving plate, markers on the patient anatomy of a person, a computed geometric boundary of the X-ray projection field, and/or optical tags on the patient anatomy of the person that determine where the patient anatomy is located in a 3D coordinate system of the AR headset. This means the X-ray generated image may be scaled appropriately based on known geometric attributes of the imaging device and/or measurable aspects of the patient being imaged (e.g., anatomy).

The scale and position of the X-ray generated image can be modified to more accurately represent the projection distortions that may be present at that measured or geometric point in the X-ray projection field. This can correct for projection minification or magnification in the X-ray generated image. More specifically, as the X-ray generated image moves away from the X-ray source, the X-ray generated image should be magnified to match the size of real objects nearer to that position. Otherwise, the X-ray generated image, which is a projection of the human anatomy in the X-ray projection field, will be too small (or large) to match the real human anatomy. Conversely, as the X-ray generated is moved toward the X-ray source, the image scaling can be decreased.

The reason for the image scaling or image magnifications and minifications are because X-ray generated images have projection distortions that can be addressed through minification or magnification prior to displaying the image next to the anatomy of interest. For example, one may think of X-ray source as a point source. If an object such as a ball is in the X-ray, the same ball at different depths from the X-ray source results in a different sized projection. When the ball is closer to the X-ray source the ball looks bigger than the ball's actual size. In order to be able to adjust for projection distortion, the distance or focal length between the X-ray source and the X-ray detector plus the resolution of the plate can be used. Anatomy that is near the X-ray detector or X-ray plate can be displayed on an X-ray generated image near the true anatomical size. However, if the anatomy is at a shallower depth (e.g., higher up) in the X-ray projection field, then the user can pinch and drag the X-ray generated image and move the X-ray generated image toward the X-ray source and the whole image may be scaled down in size, as viewed in the AR headset.

In order for the scaling to work well, calibration may be obtained for the C-arm or other X-ray imaging device being used. More specifically, the distance between the X-ray detector and the X-ray source can be measured and the size of the X-ray detection plate may be known. Alternatively, X-ray opaque objects such as balls or geometric shapes may be used to calibrate the scaling of the X-ray images. In yet another alternative, if enough geometric information is known about the X-ray imaging device, the distance between the X-ray source and X-ray detector may be calculated.

Another way of calibrating for the imaging device may be use of the optical markers or optical tags to determine known geometry of the imaging device. The resolution of the X-ray detector plate may also be known in advance and the resolution of the X-ray detector plate generally stays the same. Knowing the geometry of the imaging device and the X-ray detector plate may also enable computing of the focal distance. The resolution of the plate, the focal distance, size and shape of the projection field (e.g., radiation pyramid or radiation cone) can all be measured or determined.

This technique may also be used for portable, hand-held X-ray guns where the distance between the X-ray emitter and X-ray detector is known. In this example, the X-ray generated images may linked to the anatomy of the person using optical codes too. When the portable X-ray gun is moved away, then the X-ray generated image may remain as an overlay on the anatomy of the person.

In another example, an arm or leg may be held at some arbitrary distance over an X-ray detector. The X-ray generated image that will be shown on the AR headset from what the detector plate captures may be shown smaller than the actual arm viewed through AR headset lenses. A user may then pinch and drag up on the X-ray generated image viewed through the AR headset and then the system can size the X-ray generated image to the actual size or true size of the anatomy or arm viewed in the AR headset. If there are two different structures that the image is desired to match, the user can drag one portion of the X-ray generated image to the size of the structure being matched, and then the user may drag a second portion of the X-ray generated image to a different depth in the X-ray projection field and each structure in the X-ray generated image may be at or near the actual size of the desired anatomy. In this situation, the X-ray generated image may end up at an oblique angle to the X-ray source and X-ray detector. Due to being able select and move points on the X-ray generated image, the user can pick the scaling that is wanted for different anatomical structures.

In one configuration, the X-ray generated image may scale pixels based on their depth in the projection field formed by the X-ray projection field. This allows the user to match the X-ray generated image to the actual size of the anatomical structure(s) of interest. Accordingly, the X-ray generated image may be scaled differently at different edges. The X-ray generated image that is a 2D image can be scaled orthogonally to the orientation of the X-rays along the pyramid or the X-ray generated image can be scaled obliquely in the pyramid. This allows the anatomical size of the X-ray generated image to be matched with the real anatomy as viewed through the AR headset. If a medical professional has a person's knee in C-arm and the knee is angled down, then the X-ray generated image can be modified to match the size of the knee anatomy as the knee slopes in the X-ray projection field.

Optical tags (e.g., AprilTags) on the person's anatomy, such as an arm or leg, can also be used for sizing of the X-ray generated. The size of the optical tags is known in advance. For example, the optical tag maybe 1 cm or 2 cm square. Identifying the optical tag with the AR headset also identifies where the surface or skin of the part of the body is located. The X-ray generated image depth can be matched to a surface layer of the anatomy or the X-ray generated image may be set to be a selected depth near or under the optical tag.

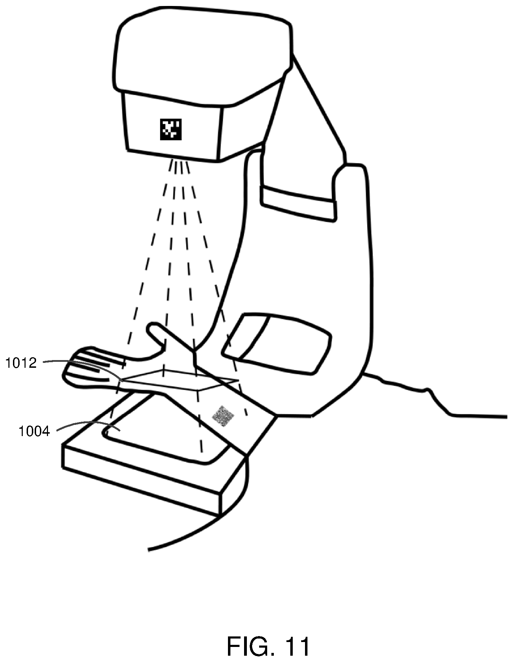

FIG. 11 illustrates an example of the X-ray generated image 1012 which has been moved and scaled, as compared to FIG. 10, to match an anatomy scale of the patient anatomy at a point nearer to the 1004 X-ray detector plate, as viewed through the AR headset. In this case, the X-ray generated image 1012 has been magnified. If the X-ray generated image is moved to a point where it appears to be in the middle of an anatomical structure (e.g., in the middle of an arm of a patient), then the X-ray generated image can be scaled up and the scaling may match the actual size of the arm due to scaling the X-ray generated image using the geometric attributes of the X-ray device. Thus, the X-ray generated image may have a position and scale of the X-ray generated image modified to correspond to the position and scale of the patient anatomy viewed through the AR headset. The means the X-ray generated image can be adjusted in size to be similar to or match the size of the anatomy being imaged. More specifically, the X-ray generated image 1012 may be overlaid at a location such as: a location intersecting a body part of a person, a location adjacent to anatomy of interest, a location in proximity to skin of the person, a location aligned with an angle of disposition of the patient anatomy in the person, a location at a center of mass of the body part in at least one axis, or a location bisecting anatomy of the anatomy of the person in at least one axis.

In another example, the position and orientation of the X-ray generated image may be set to a position and orientation of an expected path of a medical instrument through the patient anatomy during a medical procedure. If a needle is to travel along a certain plane or to a certain point in the patient's real anatomy, then the X-ray generated image may be placed in the 3D coordinate system of the AR headset so that a medical professional can follow the plane shown by the X-ray generated image while performing a medical procedure.

As described earlier, one or more 2D images may be linked to a portion of human anatomy and when the patient anatomy moves, the 2D images can move with the patient anatomy while maintaining their relative position and orientation with respect to the patient's anatomy. The X-ray generated image can move when the patient anatomy moves while maintaining a reference placement with respect to the patient anatomy. To make this happen, a marker with an image visible marker can be identified on the anatomy of a person from which the X-ray generated image is obtained. The marker may be an optical code, a visual marker, a colored visual marker, metal marker, an IR reflector, IR marker or some other type of visible marker. The AR headset may then determine that the anatomy is moving and/or is outside the X-ray projection field by detecting that the marker is moving. The 2D X-ray generated images can then be moved and re-aligned to the moving anatomy by using the marker as referenced to an image visible maker in the X-ray generated image and the AR headset. For any location the patient's anatomy moves to, (e.g., the patient's arm or leg may move) the 2D X-ray generated images can be moved and maintain their original orientation and position to the patient's anatomy as defined by the projections used to capture the 2D X-ray generated images using the medical imaging device. The position modifications made to the 2D X-ray generated image by the user with respect to the patient's body may also be maintained.

As discussed, after a patient has taken the user's arm off the X-ray device, the user can still see the X-ray generated image away from the x-ray device while using the AR headset. At this point, modifications can still be made to positioning and orientation of the 2D X-ray generated image. Even when the patient's anatomical body part (such as the arm) is away from the X-ray device, a user may drag or adjust the depth of the X-ray generated image, so the user can drag the 2D X-ray generated image through the arm to any depth when the patient is on the operating table. This is performed by creating a virtual C-arm or virtual X-ray space that is away from the X-ray device and this virtual X-ray space is useable during the medical procedure. Any time a 2D X-ray generated image is moved, the virtual X-ray space may be moved with the X-ray generated image.

Each of the plurality of 2D X-ray generated images captured using the X-ray device may be a non-parallel projection or non-parallel spatial placements. Each of the plurality of X-ray generated images can be positioned with respect to each other based on initial spatial placements for each X-ray generated image with respect to the patient anatomy. Individual X-ray generated images can be linked to: a virtual marker on the common patient anatomy of the X-ray generated images, an optical code on the patient anatomy, a morphometric model of anatomy of a person or a virtual model of anatomy of the person.

One useful result of this technology is that using 2D X-ray generated images organized as a 3D spatial mapping or 3D navigation reference is possible may avoid additional and more expensive imaging like a CT scan or MRI prior to a medical procedure occurring. As a result, this technology provides augmented reality (AR) views using widely available 2D X-ray generated images. Similarly, any optical tags and image visible tags needed to be used can be put on a body of a person right before the operation. This means that an additional, non-parallel imaging session may not be needed and only one imaging session may be provided prior to a medical procedure.

Furthermore, the 2D X-ray generated images and the X-ray pyramid can be merged with any other imaging modality that may be available. Examples of other imaging modalities that may be merged with the present technology may be an MRI (magnetic resonance imaging) modality, CT (computed tomography) scanning modality, Positron Emission Tomography (PET) modality, an ultrasound modality, a fluorescence modality, an Infrared Thermography (IRT) modality, 3D Mammography, or a Single-Photon Emission Computed Tomography (SPECT) scan modality, etc. An image data set from another image modality can be aligned to the body of the person using the optical code, image visible marker and/or imaging of the image visible marker that is visible in the X-ray generated image.

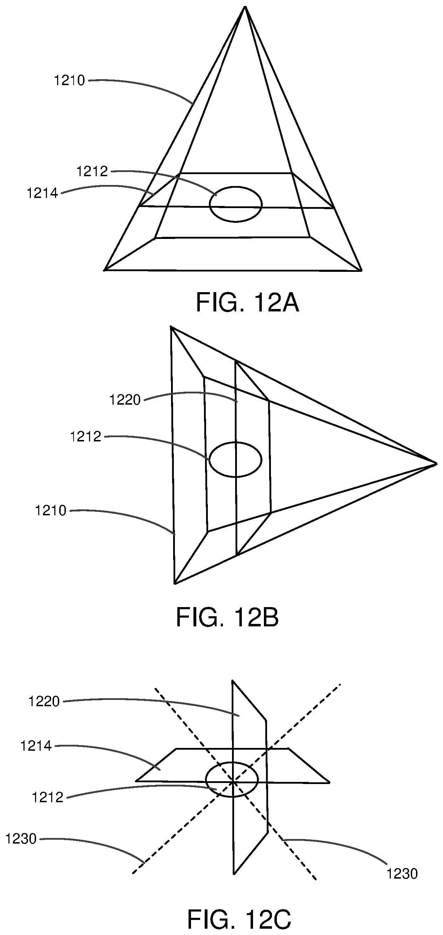

FIG. 12A illustrates that a first 2D X-ray generated image 1214 can be a projection through an anatomical structure 1212, such as a person's arm. The pyramid in FIG. 12A represents the X-ray radiation spread or projection field from an X-ray source. The zoom of the first 2D X-ray generated image 1214 can be set to bisect the anatomical structure 1212.

FIG. 12B illustrates that a second 2D image 1220 may be captured of the anatomical structure 1212. The second 2D X-ray image 1220 in the second orientation can be linked to the person's arm position and orientation using an optical tag or the shape of the arm. The projection fields or cones of FIGS 12A and 12B can be set as a virtual cone for each projection. In addition, the X-ray generated image views may be separately displayed based on which way the arm is oriented. Thus, an X-ray generated image may only be turned on when the X-ray generated image is the closest image to the medical professional. It may appear through the AR head set that a user can "look through" the arm at several angles and each X-ray generated image may be at the true size of the patient's anatomy. Because the X-ray generated images are associated with optical codes, the X-ray generated images may appear as though the images are attached to the arm and are co-registered to the correct anatomy when the arm moves.

As discussed earlier, the markers or optical codes can link the images and virtual cones or virtual pyramids to the person being imaged. The one or more of the virtual pyramids and the X-ray generated images can moved away to the operating table. The X-ray generated images may then maintain the position, orientation and scaling that were originally captured with respect to the anatomical structure.

FIG. 12C illustrates when the first X-ray generated image 1214 and the second X-ray generated image 1220 are superimposed and displayed together over the anatomical structure 1212. The dotted lines 1230 illustrate that additional 2D X-ray generated images can be captured at any angle and can be superimposed over the anatomical structure 212 thus creating additional guide layers for the 3D spatial mapping.

In a medical procedure example, a medical professional may be operating on a femur of a patient. The doctor may desire to put a rod into a femur at a certain a certain level in the bone due to a fracture. It can also be important for the doctor to miss certain items that may cause complications in the procedure. Accordingly, the projection field (e.g., any geometrically shaped radiation field) may be established and an instrument tracker may be attached to a needle or screw. The anatomical area of interest is the path for the screw or a needle. As a result, the X-ray generated image may be shown at the plane of interest or the desired path for the medical device. The doctor can see the depths and trajectories needed and then line up the tool with those trajectories and depths. In addition, the doctor may be able to see the correct relative sizes of the anatomy with the 3D guidance being seen. Further, as the doctor moves the tool up and down, the X-ray generated image can be referenced to give the doctor the correct relative sizes of anatomical structures where the tool is currently positioned.

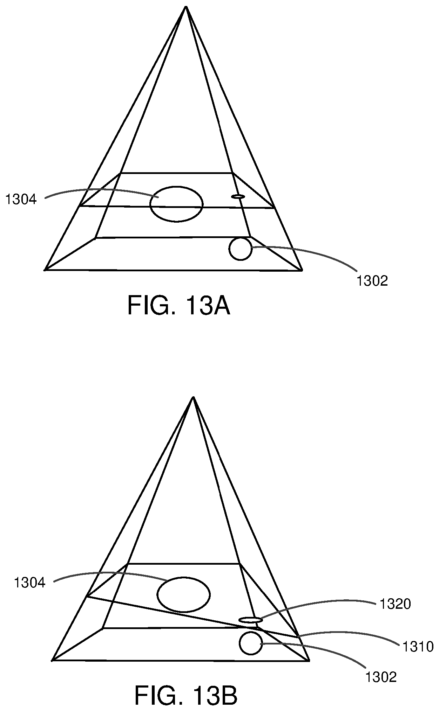

FIG. 13A illustrates how a first object 1302 may appear smaller in a 2D X-ray image when the image has been minified to match the size of a second object 1304. However, FIG. 13B illustrates that a corner 1310 may be moved down in the X-ray projection field or cone and the portion of the image that captured the first object 1302 may be magnified. Thus, each object may be viewed at nearer to 1320 or at their actual size in the image. Moving a single corner is illustrated in FIG. 13B but each corner of an image or any point on an image could be selected for movement. Continuity between that point and the other points in the can be maintained for the 2D plane by interpolating the amount of movement applied to the point moving and other points. This may result in the 2D plane of the 2D X-ray generated image being doubly oblique or other non-orthogonal orientations. For example, if the anatomy a medical profession is interested in is not parallel to the X-ray detector plate then the plane of the 2D X-ray generated image may be aligned with the orientation of anatomy of interest.

This technology provides the ability to navigate a patient's anatomy with a medical tool or other tools in 3D using two or more 2D X-ray generated images. The X-ray generated images may be displayed in an AR headset as intersecting planes which provide a 3D navigation reference.

When the 2D X-ray generated images intersect (e.g., orthogonal intersection view) using a line defined by common anatomical points, a medical professional can navigate an instrument along the virtual line. The graphical interface of the AR headset may use colors to show whether a medical instrument is lined up with a virtual guidance line. For example, a blue line can be shown as coming out of the real or virtual part of the instrument. Then when the medical instrument is lined up with a yellow virtual guidance line then a green indicator may be displayed if the doctor is lined up. Other arbitrary color schemes may also be shown for accurate alignments. The instrument guidance interface can also show a doctor where the medical tool lines up on the break out views in the bottom of the AR headset interface. They are navigational views. For example, coronal and sagittal views. On the breakout views in the 3D coordinate system there may be the virtual line that the doctor created and this can be used as a needle guidance line. The doctor can also see depth in these navigational views which can be hard to see. The doctor can see if the instrument is lined up with the virtual tracker. This guidance for the doctor prompts them to move the instrument correctly.

In the past, a doctor without guidance would use a medical tool, such as a needle, on a body of a patient but the medical tool would not necessarily be near the target the medical professional desires to hit. So, a doctor could use trial and error to line up the medical tool with the desired anatomical structure. This generally resulted in a medical tool or tip of a tool being off course or off target. However, using two or more X-ray generated images to find a point or a virtual line as a 3D track, using an AR headset that guides a doctor, can increase the accuracy of medical procedures dramatically. This technology enables a doctor to identify a point or plan a track in each plane independently. Thus, this technology can be a very accurate guide to a point or a line used by the medical professional.

In another example, two X-ray generated images of a femur may be obtained. The target for the medical procedure may be the marrow space in a femur of the patient. A doctor can mark two spots on a first X-ray generated image representing the marrow space in the femur, and this results in one virtual line. The marking can be independently repeated on a second X-ray generated image to provide a second virtual line on common anatomical structure. Those two lines can intersect and provide an intersection for both the X-ray generated images. This line can define a virtual navigation path through the 3D coordinate space viewed by the VR headset. A 3D spatial mapping is defined using the two 2D X-rays, and the doctor can view a defined trajectory in the 3D coordinates of the AR headset.

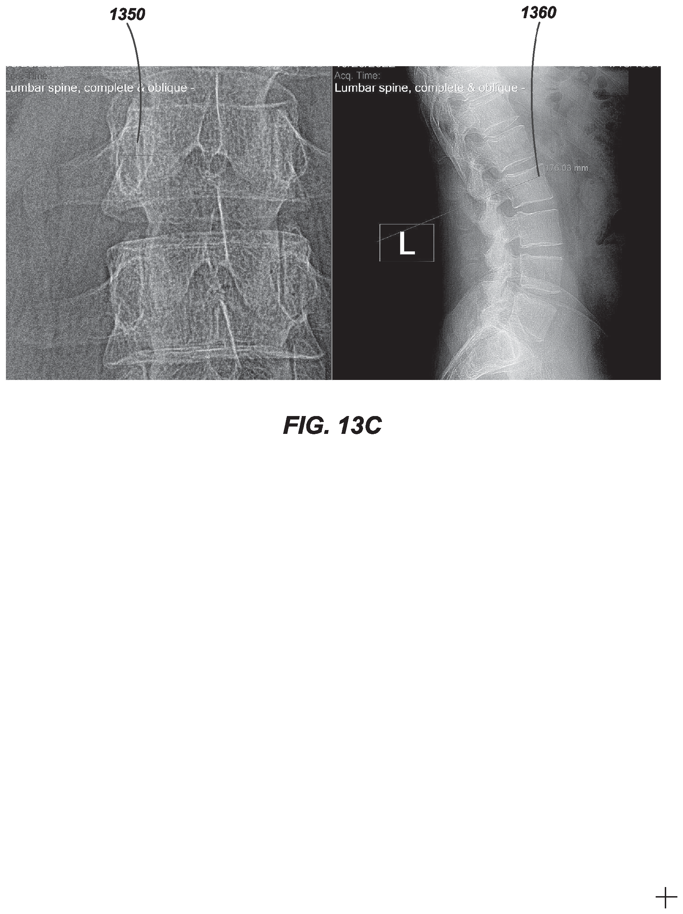

In another example illustrated in FIG. 13C, the use of one line and a point in two X-ray generated images can be used in pedicle placement. From the first view on the right, a doctor sees a channel 1350 in the end of the screw to enable screwing of the screw into the spine and marks channel with a first point. From the second view that is orthogonal to the top view, a doctor would see the shaft of the pedicle screw is desired to be placed. The doctor can draw a line 1360 on the side view regarding where the screw should be threaded into the spine. From the first view, one point is all that is needed. A line virtual may be generated between the X-ray source and the first point. This means just three points in space can be marked for navigation purposes. Two points can be defined explicitly on the second view X-ray generated image and one point can be defined on the first view X-ray generated image and another point can be implicitly defined from the X-ray source. If the first view is perpendicular to or orthogonal to the second X-ray generated image, then a line can be formed from the X-ray source to the marked point. These lines can align and the path for the pedicle screw is then defined. Since, the third point does not provide a specific depth in space, the doctor may visually define a depth on the line that is created using the third point and the X-ray source. This configuration avoids the need for a CT scan before surgery because a doctor has identified the virtual target with this process. Where the point defines a line to the X-ray source and the second image has a line, a doctor can set the depth for the medical device (e.g., screw, probe, needle, etc.) on the virtual line that is created using the point.

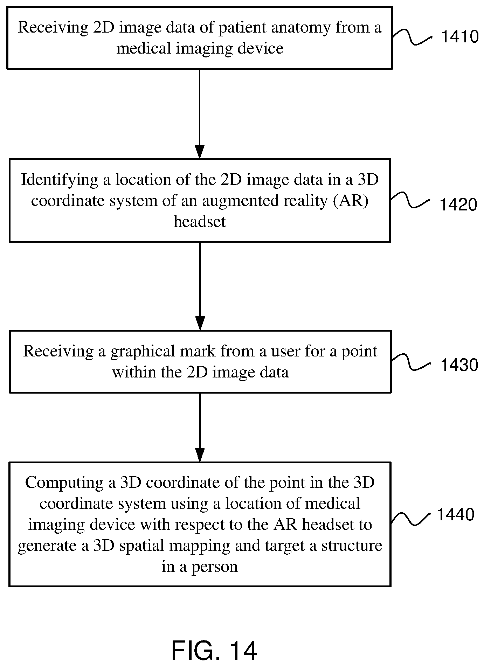

FIG. 14 is a flow chart illustrating an example method for creating a 3D spatial mapping or 3D spatial guide. One operation in the method may be receiving 2D image data of patient anatomy from a medical imaging device, as in block 1410. In one example, the 2D image data is an ultrasound image.

Another operation may be identifying a location of the 2D image data in a 3D coordinate system of an augmented reality (AR) headset, as in block 1420. The location of the imaging device may be identified by registering markers or optical codes on the imaging device (e.g., an ultrasound imaging device). Alternatively, a shape of the ultrasound imaging device may be identified and this may identify the placement (i.e., position and orientation) of the ultrasound imaging device.

A graphical mark may be received from a user for a point within the 2D image data, as in block 1430. The graphical mark may have a depth associated with the point as reported with an ultrasonic image or by an ultrasonic transducer.

A 3D coordinate of the point in the 3D coordinate system can be computed using a location of medical imaging device with respect to the AR headset to generate a 3D spatial mapping and target a structure in a person, as in block 1440. The 2D image(s) obtained from the medical imaging device may be displayed while viewing patient anatomy through an augmented reality (AR) headset. In addition, a procedure guidance indicator (i.e., a virtual line or arrow) from a medical tool identified by the AR headset may be displayed to the 3D coordinate of the point.

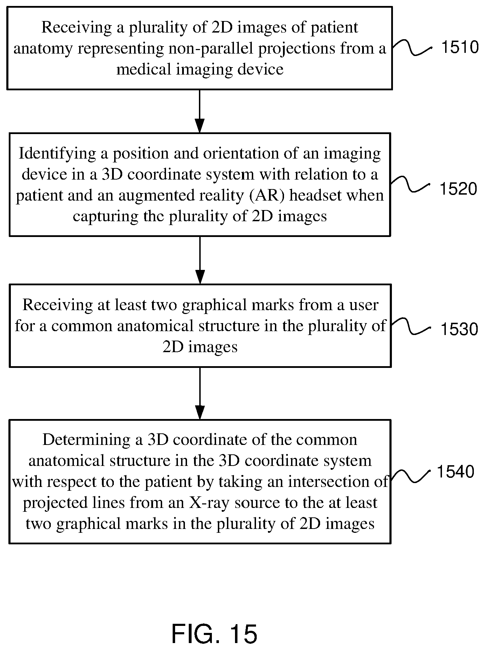

FIG. 15 illustrates an example of a method for 3D spatial mapping. One operation in the method may be receiving a plurality of 2D images of patient anatomy representing non-parallel projections from the medical imaging device, as in block 1510. The 3D images may be at least one of: X-ray generated images, ultrasound images, CT generated images, MRI generated images or other 2D medical images.

A position and orientation of an imaging device in a 3D coordinate system may be identified with relation to a patient and an augmented reality (AR) headset when capturing the plurality of 2D images, as in block 1520. At least two graphical marks may be received from a user for a common anatomical structure in in the plurality of 2D images, as in block 1530. The graphical mark may be at least one of a point, line, navigational track, or 3D target.

A 3D coordinate of the common anatomical structure in the 3D coordinate system may be determined in the 3D coordinate system with respect to the patient by taking an intersection of projected lines from an X-ray source to the at least two graphical marks in the plurality of 2D images, as in block 1540. The 2D images may be aligned by using a virtual line placed on common anatomy in each of the 2D images.

The 2D images from the medical imaging device can be displayed while viewing patient anatomy using an augmented reality (AR) headset, in order to generate a 3D spatial mapping. A navigation path from a medical tool identified by the AR headset to the 3D coordinate of the common anatomical structure may also be provided.

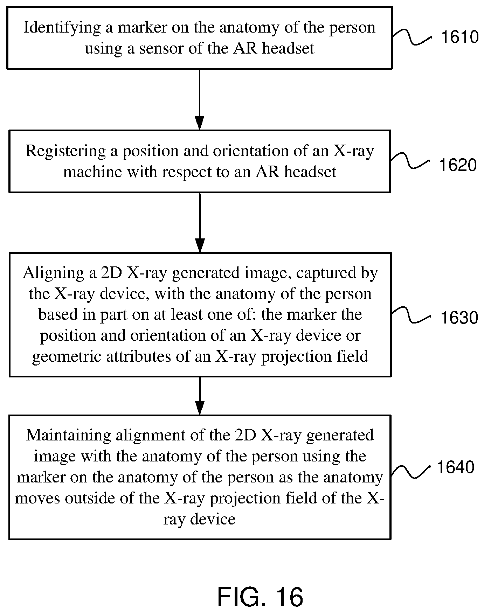

FIG. 16 illustrates a method for aligning an x-ray generated image with anatomy of a person using an augmented reality (AR) headset. One operation in the method is identifying a marker on the anatomy of the person using a sensor of the AR headset, as in block 1610. A position and orientation of an X-ray device in a 3D coordinate system may be registered with respect to an AR headset, as in block 1620.

A 2D X-ray generated image, captured by the X-ray device, may be aligned with the anatomy of the person. The alignment may use at least one of: the marker, the position and orientation of an X-ray device or geometric attributes of an X-ray projection field, as in block 1630.

A further operation in the method may be maintaining alignment of the 2D X-ray generated image with the anatomy of the person using the marker on the anatomy of the person as the anatomy moves outside of the X-ray projection field of the X-ray device, as in block 1640. For example, the anatomy may move outside the X-ray projection field with the marker. Accordingly, the X-ray generated image may be re-aligned with the anatomy of the person using the marker on the anatomy of the person. The marker may enable detection of an orientation of the anatomy of the person in a 3D coordinate system of the AR headset. The X-ray generated image can be aligned to the orientation of the anatomy by using a visible marker and orientation of the anatomy in the X-ray generated image using the AR headset.

Some of the functional units described in this specification may have been labeled as modules, in order to more particularly emphasize their implementation independence. For example, a module may be implemented as a hardware circuit comprising custom VLSI circuits or gate arrays, off-the-shelf semiconductors such as logic chips, transistors, or other discrete components. A module may also be implemented in programmable hardware devices such as field programmable gate arrays, programmable array logic, programmable logic devices or the like.

Modules may also be implemented in software for execution by various types of processors. An identified module of executable code may, for instance, comprise one or more blocks of computer instructions, which may be organized as an object, procedure, or function. Nevertheless, the executables of an identified module need not be physically located together, but may comprise disparate instructions stored in different locations which comprise the module and achieve the stated purpose for the module when joined logically together.

Indeed, a module of executable code may be a single instruction, or many instructions, and may even be distributed over several different code segments, among different programs, and across several memory devices. Similarly, operational data may be identified and illustrated herein within modules, and may be embodied in any suitable form and organized within any suitable type of data structure. The operational data may be collected as a single data set, or may be distributed over different locations including over different storage devices. The modules may be passive or active, including agents operable to perform desired functions.

The technology described here can also be stored on a computer readable storage medium that includes volatile and non-volatile, removable and non-removable media implemented with any technology for the storage of information such as computer readable instructions, data structures, program modules, or other data. Computer readable storage media include, but is not limited to, RAM, ROM, EEPROM, flash memory or other memory technology, CD-ROM, digital versatile disks (DVD) or other optical storage, magnetic cassettes, magnetic tapes, magnetic disk storage or other magnetic storage devices, or any other computer storage medium which can be used to store the desired information and described technology.

The devices described herein may also contain communication connections or networking apparatus and networking connections that allow the devices to communicate with other devices. Communication connections are an example of communication media. Communication media typically embodies computer readable instructions, data structures, program modules and other data in a modulated data signal such as a carrier wave or other transport mechanism and includes any information delivery media. A "modulated data signal" means a signal that has one or more of its characteristics set or changed in such a manner as to encode information in the signal. By way of example, and not limitation, communication media includes wired media such as a wired network or direct-wired connection, and wireless media such as acoustic, radio frequency, infrared, and other wireless media. The term computer readable media as used herein includes communication media.

Furthermore, the described features, structures, or characteristics may be combined in any suitable manner in one or more examples. In the preceding description, numerous specific details were provided, such as examples of various configurations to provide a thorough understanding of examples of the described technology. One skilled in the relevant art will recognize, however, that the technology can be practiced without one or more of the specific details, or with other methods, components, devices, etc. In other instances, well-known structures or operations are not shown or described in detail to avoid obscuring aspects of the technology.

Although the subject matter has been described in language specific to structural features and/or operations, it is to be understood that the subject matter defined in the appended claims is not necessarily limited to the specific features and operations described above. Rather, the specific features and acts described above are disclosed as example forms of implementing the claims. Numerous modifications and alternative arrangements can be devised without departing from the spirit and scope of the described technology.

## Claims

1. A system for adjusting an X-ray generated image with respect to patient anatomy, the system comprising:
an augmented reality (AR) headset comprising a display;
a processor communicatively coupled to the AR headset; and
a memory storing instructions which, when executed by the processor, cause the system to:
register a position and orientation of an X-ray device in a 3D coordinate space;
control the display of the AR headset to overlay an X-ray generated image, as captured by the X-ray device, at the position and orientation associated with an X-ray projection field as defined in part by the position and orientation of the X-ray device and geometric attributes of the X-ray projection field;
receiving, from a user of the AR headset, an identification of a 3D point of interest in the X-ray projection field; and
positioning and scale the X-ray generated image within an X-ray projection field to include the 3D point of interest.

2. The system as in claim 1, wherein the instructions for positioning and scaling the X-ray generated image cause the image to be scaled to match an anatomy scale of the patient anatomy at the 3D point of interest, as viewed through the AR headset.

3. The system as in claim 1, wherein the instructions further cause the system to modify a position and scale of the X-ray generated image to correspond to a position and scale of the patient anatomy as viewed through the AR headset.

4. The system as in claim 3, wherein the instructions for modifying the position and scale of the X-ray generated image further cause the system to overlay the x-ray generated image at a location that is at least one of: a location intersecting a body part of a person, a location adjacent to anatomy of interest, a location in proximity to skin of the person, a location aligned with an angle of disposition of the patient anatomy in the person, or a location at a center of mass of the body part in one axis, or a location bisecting anatomy of the anatomy of the person in one axis.

5. The system as in claim 1, wherein the instructions cause the system to set the position and orientation of the X-ray generated image to represent an expected path of a medical instrument through the patient anatomy during a medical procedure.

6. The system as in claim 1, wherein the instructions further cause the system toreceive a selection of at least one point in the X-ray generated image from a user; and
receive a dragging instruction for the X-ray generated image in order to move the X-ray generated image.

7. The system as in claim 1, wherein the geometry of the X-ray projection field is defined using a resolution of a detector array and a distance between an X-ray emitter and the detector array.

8. The system as in claim 1, wherein the instructions further cause the system to identify a marker with an image visible marker on anatomy of a person from which the X-ray generated image is obtained; and
align the X-ray generated image to the anatomy by using the marker as referenced to an image visible maker in the X-ray generated image, using the AR headset.

9. The system as in claim 8, wherein the marker is an optical code, infrared reflector, or a visible marker.

10. The system as in claim 1, wherein the instructions further cause the system to capture a plurality of X-ray generated images from the X-ray device, wherein the patient anatomy is at non-parallel projections during capture of each of the X-ray generated images; and
position the plurality of X-ray generated images with respect to on another based on initial projections of each X-ray generated image with respect to the patient anatomy.

11. The system as in claim 1, wherein the instructions further cause the system to move the X-ray generated image when the patient anatomy moves while maintaining a projection position and orientation with respect to the patient anatomy; or wherein the X-ray generated image is linked to at least one of: a marker on the patient anatomy, an optical code on the patient anatomy, a morphometric model of anatomy of a person or a virtual model of anatomy of the person.

12. The system as in claim 1, wherein the instructions further cause the system toreceive an identification of a 3D point for a corner of the X-ray generated image from the user;
adjust a position of the corner of the X-ray generated image with respect to the X-ray device to include the 3D point; and
set magnification or minification of portions of the X-ray generated image based on locations of the corner of the X-ray generated image with respect to a detector array of the X-ray device.

13. A system for aligning an X-ray generated image with anatomy of a person, the system comprising
a sensor and a display, a processor communicatively coupled to the AR headset; and a memory storing instructions which, when executed by the processor, cause the system to
identify a marker on the anatomy of the person using a sensor of the AR headset;
register a position and orientation of an X-ray device in a 3D coordinate system;
align a 2D X-ray generated image, captured by the X-ray device, with the anatomy of the person based in part on at least one of: the marker, the position and orientation of an X-ray device or geometric attributes of an X-ray projection field; and
align the 2D X-ray generated image with the anatomy of the person using the marker on the anatomy of the person as in order to maintain alignment outside of the X-ray projection field of the X-ray device.

14. The system as in claim 13, wherein the instructions for maintaining alignment further cause the system to detect the anatomy has moved outside the X-ray radiation field using the marker; and
re-align the X-ray generated image with the anatomy of the person using the marker and image visible marker on the anatomy of the person to maintain the X-ray generated image orientation with the anatomy.

15. The system as in claim 13, further comprising instructions which, when executed by the processor, cause the system to scale the X-ray generated image when the X-ray generated image has moved away from X-ray device by using geometric attributes of a projection field stored with the X-ray generated image.
